# FASCICULE DE BREVET EUROPEEN

(11) **EP 3 063 166 B1**
(45) Date de publication et mention de la délivrance du brevet: **06.02.2019**
(21) Numéro de dépôt: 14824893.3
(22) Date de dépôt: 28.10.2014
(51) Int. Cl.: C07J 5/00, C07C 49/637, C07C 21/00

(54) **PROCEDE DE PREPARATION DE DERIVES STEROIDIENS 6-ALKYLÉS ET LES 5,6,7,8-TETRAHYDRONAPHTHALÈNE-2(4ALPHA.H)-ONES ALKYLÉS CORRESPONDANTS**
VERFAHREN ZUR HERSTELLUNG VON 6-ALKYLIERTEN STEROIDDERIVATEN UND VON DEN ENTSPRECHENDEN ALKYLIERTEN 5,6,7,8-TETRAHYDRONAPHTHALEN-2(4 ALPHA.H)-ONEN
METHOD FOR PREPARING 6-ALKYLATED STEROIDAL DERIVATIVES AND CORRESPONDING ALKYLATED 5,6,7,8-TETRAHYDRONAPHTHALENE-2(4 ALPHA.H)-ONES

(30) Priorité: 28.10.2013 FR 1360498
(43) Date de publication de la demande: 07.09.2016
(73) Titulaire: SANOFI, 75008 Paris (FR)
(72) Inventeur: DUEZ, Stéphanie, 81679 Munich (DE); QUINIO, Pauline, 63739 Aschaffenburg (DE); HAESSLEIN, Jean-Luc, 75008 Paris (FR); LHERMITTE, Frédéric, 75008 Paris (FR)
(74) Mandataire: Sanofi
(86) Numéro de dépôt international: PCT/FR2014/052740
(87) Numéro de publication internationale: WO 2015/063408

(56) Documents cités:
- DE-B- 1 059 906
- GB-A- 926 472
- US-A- 2 816 902
- US-A- 2 908 696
- US-A- 2 959 602
- JOHN H. FRIED ET AL: "Alkylated Adrenal Hormones. The Synthesis of 6[alpha]-Methyl Cortical Steroids", JOURNAL OF THE AMERICAN CHEMICAL SOCIETY, vol. 81, no. 5, 1 mars 1959 (1959-03-01), pages 1235-1239, XP055124850, ISSN: 0002-7863, DOI: 10.1021/ja01514a055
- ANNEN K ET AL: "17-PIVALATE IN DER PREGNANREIHE//17-PIVALATE DERIVATIVES OF PREGNANE", LIEBIGS ANNALEN DER CHEMIE, VERLAG CHEMIE GMBH. WEINHEIM, DE, vol. 4, 1 janvier 1983 (1983-01-01), pages 705-711, XP001119196, ISSN: 0170-2041
- VOLKERS A A ET AL: "Alkylations of tricyclo[5.2.1.0<2,6>]deca-4,8-dien-3-one by a cuprate reaction", TETRAHEDRON, ELSEVIER SCIENCE PUBLISHERS, AMSTERDAM, NL, vol. 65, no. 1, 3 janvier 2009 (2009-01-03), pages 389-395, XP025712261, ISSN: 0040-4020, DOI: 10.1016/J.TET.2008.10.028 [extrait le 2008-10-17]
- LEO A. PAQUETTE ET AL: "Applications of the Squarate Ester Cascade to the Expeditious Synthesis of Hypnophilin, Coriolin, and Ceratopicanol", JOURNAL OF THE AMERICAN CHEMICAL SOCIETY, vol. 124, no. 31, 1 août 2002 (2002-08-01) , pages 9199-9203, XP055124726, ISSN: 0002-7863, DOI: 10.1021/ja020474t
- H. J. RINGOLD ET AL: "STEROIDS. CXXVII. 1 6-HALO PROGESTATIONAL AGENTS", JOURNAL OF THE AMERICAN CHEMICAL SOCIETY, vol. 81, no. 13, 1 juillet 1959 (1959-07-01), pages 3485-3486, XP055125053, ISSN: 0002-7863, DOI: 10.1021/ja01522a090
- DATABASE CA [Online] CHEMICAL ABSTRACTS SERVICE, COLUMBUS, OHIO, US; LI, NAN ET AL: "Synthesis of 11,17,21-triacetoxy-6.alpha.-fluoro-pregn- 1,4-diene- 3,20-dione", XP002739461, extrait de STN Database accession no. 2010:1382659 & LI, NAN ET AL: "Synthesis of 11,17,21-triacetoxy-6.alpha.-fluoro-pregn- 1,4-diene- 3,20-dione", HUAXUE YU SHENGWU GONGCHENG , 27(8), 42-43, 47 CODEN: HYSGAF; ISSN: 1672-5425, 2010,
- LAL G SANKAR: "Site-selective fluorination of organic compounds using 1-alkyl-4-fluoro-1,4-diazabicyclo(2.2.2)oc tane salts (selectfluor reagents)", THE JOURNAL OF ORGANIC CHEMISTRY, AMERICAN CHEMICAL SOCIETY, US, vol. 58, no. 10, 1 janvier 1993 (1993-01-01), pages 2791-2796, XP002299135, ISSN: 0022-3263, DOI: 10.1021/JO00062A023

## Description

La présente invention a pour objet un procédé d'alkylation de composés de formule (III) :

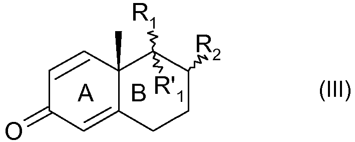

Pour donner des composés de formule (I) 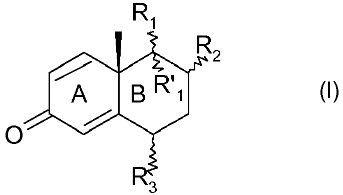
Où R₁, R₂ représentent indépendamment l'un de l'autre un atome d'hydrogène, un radical alkyle comprenant de 1 à 3 atomes de carbone, ou un atome d'halogène, ou bien R₁ et R₂ forment ensemble un cycle carboné comprenant de 4 à 6 atomes de carbone, ortho condensé au cycle B du composé (I), ledit cycle comprenant optionnellement une ou plusieurs doubles liaisons et étant optionnellement substitué par un ou plusieurs groupement alkyle comprenant 1 à 3 atomes de carbone, ou bien R₁ et R₂ forment ensemble les cycles C et D d'un squelette carboné stéroïdien, lesdits cycles C et D comprenant optionnellement une ou plusieurs doubles liaisons et étant optionnellement substitués par un ou plusieurs groupes choisis parmi les groupes alkyles linéaires ou ramifiés comprenant de 1 à 12 atomes de carbone, les groupes acyles comprenant de 1 à 12 atomes de carbone et optionnellement substitués par un ou plusieurs groupe hydroxyles, les groupements carboxyle, hydroxyle, ou oxo, sous forme libre ou protégée, ou les atomes d'halogènes, préférentiellement le fluor, chaque position desdits cycles C et D pouvant porter un ou, lorsque cela est possible, deux substituants,
R'₁ représente un atome d'hydrogène ou d'halogène, préférentiellement le fluor, et R₁ et R'₁ sont situés de part et d'autre du plan des cycles A et B,
R₃ représente un groupement alkyle comprenant de 1 à 4 atomes de carbone, préférentiellement un groupe méthyle, préférentiellement un groupe méthyle positionné en α, 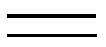 représente une simple ou une double liaison,
et 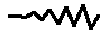 représente soit une simple liaison en alpha ou beta des cycles A et B, soit une double liaison dans le plan des cycles A et B.

En particulier, la présente invention a pour objet un procédé de préparation de 6-alkyl-3-oxo-Δ1,4-pregnadiènes ou de 6-alkyl-3-oxo-4-pregnènes, de formule (IA') ci-dessous : 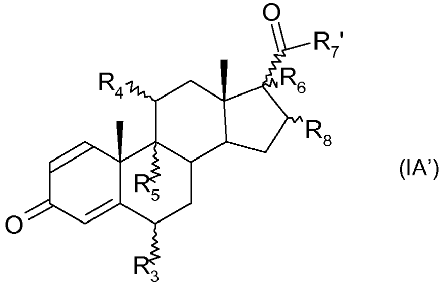
Où R₃ est un groupement alkyle comprenant de 1 à 4 atomes de carbone, préférentiellement un méthyle,
R₄ est soit l'hydrogène, soit un groupement hydroxyle ou oxo, sous forme libre ou protégée, par exemple un groupe hydroxyle sous forme estérifiée, préférentiellement sous forme acétylée, ou sous forme trifluoroacétylée,
R₅ est soit un atome d'hydrogène, soit un atome d'halogène, préférentiellement le fluor,
R₆ est soit un atome d'hydrogène, soit un groupement hydroxyle, sous forme libre ou protégée, par exemple sous forme estérifiée,
R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée, préférentiellement un groupe hydroxyméthyle sous forme acétylée, ou sous forme trifluoroacétylée,
R₈ est soit un atome d'hydrogène, soit un groupement alkyle comprenant de 1 à 3 atomes de carbone,
où les groupements -R₆ et -(CO)R₇' sont situés de part et d'autre du plan des cycles A, B, C, D,
où le groupement oxo situé sur le carbone en position 20 du squelette stéroïdien peut être sous forme libre ou protégée,
où 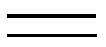 représente une simple ou une double liaison,
où 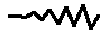 représente soit une simple liaison en alpha ou beta ou dans le plan des cycles A, B, C, D, soit, lorsque cela est possible, une double liaison dans le plan des cycles A, B, C, D.

Dans le cas des stéroïdes, l'introduction en position 6 d'un substituant alkyle, notamment méthyle, a pour effet d'augmenter l'activité ou de réduire les effets indésirables par rapport à la structure correspondante non alkylée. C'est le cas par exemple pour des agents progestatifs tels que le megestrol ou l'acétate de megestrol, la medrogestone, la medroxyprogesterone ou l'acétate de medroxyprogesterone, et de corticostéroïdes tels que la 6 α-méthylprednisolone, la medrysone, l'endrysone, la fluorométholone, le cortivasol, qui sont des séroïdes méthylés en position 6.

Les procédés d'alkylation en 6 de composés stéroïdiens de l'art antérieur ont en commun d'effectuer l'alkylation sur une structure saturée en 1-2.

En particulier, les procédés de méthylation connus passent par l'introduction d'un méthylène en 6 sur une structure saturée en 1-2.

Le méthylène peut ensuite être converti en α ou β méthyle par hydrogénation, selon la méthode décrite dans la publication « Modified Steroid Hormones, application of the Vilsmeier reaction to 11β hydroxy steroids », D Burn and J.P. Yardley, Tetrahedron, Vol 25, pp 1155-1158.

Une étape éventuelle de création de la double liaison en 1-2 est effectuée, postérieurement à l'alkylation, avec des agents déshydrogénant tels que le 2,3-dichloro-5,6 dicyano-1,4-benzoquinone, comme décrit dans le brevet GB1051613, ou bien d'autres méthodes chimiques ou microbiologiques connues, par exemple décrites dans la demande IL59161.

L'introduction du méthylène sur la structure saturée en 1-2 peut se faire comme décrit dans le brevet EP0034115, ou dans la publication « A simple Method for 6-methylation of 3-oxo-Δ4-steroids », Annen et al., SYNTHESIS, January 1982, 34-40, par réaction d'un acétal de formaldéhyde en présence de dérivés d'acide phosphorique.

Cette introduction du méthylène peut également se faire, comme décrit dans la demande WO93/00354 par une réaction de Mannich utilisant le formaldéhyde, une amine secondaire (la N-méthylaniline) et catalysée par l'acide trifluoroacétique dans le tétrahydrofurane.

J. Am. Chem. Soc. Vol 81(5) pages 1235-1239 (1959) et Liebig's Ann. Chem. pages 705-711 (1983) décrivent des procédés pour l'alkylation de la position 6 des stéroïdes, sans passer par des intermédiaires 6-halogénés. Toutefois, ces méthodes comportent un nombre important d'étapes.

Par ailleurs, dans le cas de la synthèse de composés insaturés en position 1-2 du cycle correspondant au cycle A d'un squelette stéroïdien, il peut être avantageux de procéder à l'akylation directe d'une structure possédant déjà cette insaturation, par exemple pour les stéroïdes, à l'alkylation directe d'une structure Δ1,4-pregnadiène. Ceci est par exemple intéressant pour la synthèse de la 6α-méthylprednisolone, l'endrysone, la fluorométholone.

Il existe donc un besoin pour le développement de voies simples et économiques d'introduction d'un substituant alkyle, en particulier méthyle, en position 6 de ces structures.

La présente invention a pour objet de tels procédés d'alkylation. La présente invention a en effet pour objet des procédés de préparation de composés répondant à la formule (I) ci-dessous, 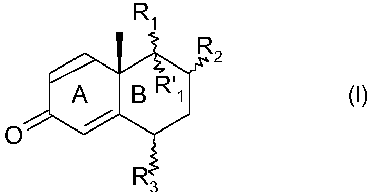
Où R₁, R₂ représentent indépendamment l'un de l'autre un atome d'hydrogène, un radical alkyle comprenant de 1 à 3 atomes de carbone, ou un atome d'halogène, ou bien R₁ et R₂ forment ensemble un cycle carboné comprenant de 4 à 6 atomes de carbone, ortho condensé au cycle B du composé (I), ledit cycle comprenant optionnellement une ou plusieurs doubles liaisons et étant optionnellement substitué par un ou plusieurs groupements alkyle comprenant 1 à 3 atomes de carbone, ou bien R₁ et R₂ forment ensemble les cycles C et D d'un squelette carboné stéroïdien, lesdits cycles C et D comprenant optionnellement une ou plusieurs doubles liaisons et étant optionnellement substitués par un ou plusieurs groupes choisis parmi les groupes alkyles linéaires ou ramifiés comprenant de 1 à 12 atomes de carbone, les groupes acyles comprenant de 1 à 12 atomes de carbone et optionnellement substitués par un ou plusieurs groupes hydroxyles, les groupements carboxyle, hydroxyle, ou oxo, sous forme libre ou protégée, ou les atomes d'halogènes, préférentiellement le fluor, chaque position desdits cycles C et D pouvant porter un ou, lorsque cela est possible, deux substituants,
R'₁ représente un atome d'hydrogène ou d'halogène, préférentiellement le fluor, et R₁ et R'₁ sont situés de part et d'autre du plan des cycles A et B,
R₃ représente un groupement alkyle comprenant de 1 à 4 atomes de carbone, préférentiellement un groupe méthyle, préférentiellement un groupe méthyle positionné en α,
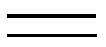 représente une simple ou une double liaison,
et 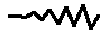 représente soit une simple liaison en alpha ou beta des cycles A et B, soit une double liaison dans le plan des cycles A et B.

Les procédés selon l'invention comprennent une étape d'alkylation d'un composé de formule (II) 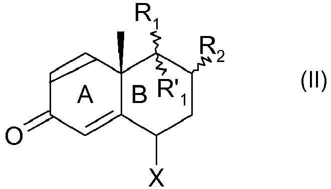
Où R₁, R'₁ et R₂, 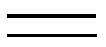 et 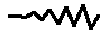 sont tels que définis dans le composé (I),
Où X est un atome d'halogène, préférentiellement le brome ou l'iode, préférentiellement le brome,
avec un agent d'alkylation organométallique permettant d'introduire un groupement alkyl R₃ en substitution de l'atome d'halogène X du composé (II), optionnellement en présence d'un catalyseur métallique, préférentiellement au palladium ou au cuivre, pour aboutir au composé de formule (I).

Le composé (II) peut être obtenu par halogénation, selon les techniques connues de l'homme du métier, d'un composé de formule (III) : 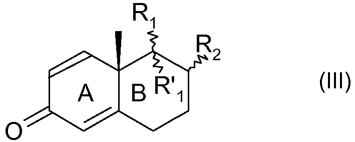 Où R₁, R'₁ et R₂, 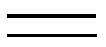 et 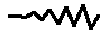 sont tels que définis précédemment

Par exemple, on peut effectuer une bromation du composé (III) par réaction avec un N-Bromoimide tel que le N-bromo-succinimide, en présence d'un initiateur radicalaire tel que le peroxyde de benzoyle ou l'AIBN (azobisisobutyronitrile), selon les conditions classiques d'une réaction de Wohl-Ziegler.

Par protection du groupe hydroxy, on entend toutes protections usuelles connues du chimiste. On peut citer par exemple les éthers clivables tels que ceux formés avec un groupe C₍₁₋₆₎ alkyle, notamment méthyle ou t-butyle, avec un groupe C₍₁₋₆₎ alkyl-phényle, notamment benzyle, p-méthoxybenzyle, p-nitrobenzyle, les éthers d'allyle, trityle, méthoxyméthyle, méthoxyethoxyméthyle, éthoxyéthyle, tétrahydropyranyle, les éthers silylés, notamment les éthers de triméthylesilyle, triéthylesilyle ou triisopropylsilyle, de t-butyldiméthylsilyle ou de diméthylarylsilyle.

On peut encore citer les esters clivables tels que par exemple ceux formés avec un groupe acétyle, benzoyle, phénylacétyle, formyle, haloacétyle, tel que choracétyle, di ou trichloracétyle, ou trifluoroacétyle. On peut encore citer les carbonates, ainsi que les cétals cycliques tels que -O-(CH₂)ₘ-O-, m étant de préférence 1, 2 ou 3.

Par protection du groupe oxo, on entend toute protection connue du chimiste et notamment les acétals, cétals et thiocétals cycliques tels que -O-(CH₂)ₘ-O-, -O-(CH₂)ₘ-S-, - S-(CH₂)ₘ-S-, -O-CH₂-C(C₁₋₄ alcoyl)₂-CH₂-O-, ou encore les cétals acycliques tels que -(CH₃O)₂- ou -(EtO)₂-, m étant de préférence 1, 2 ou 3.

Lorsque les composés (I), (II), et (III) des procédés selon l'invention comportent des groupements oxo et/ou hydroxyle voisins, ceux-ci peuvent être protégés conjointement par un même acétal, cétal ou thioacétal cyclique tels que ceux mentionnés ci-dessus.

La protection de groupements oxo et/ou hydroxyle voisins par des cétals cycliques peut aboutir également à la formation de composés oxa-spiraniques.

Par exemple, dans une variante des procédés selon l'invention, on note les composés particuliers (Id) et (lld) ci-dessous, où X représente un atome d'halogène, préférentiellement le brome ou l'iode, préférentiellement le brome, peut se trouver sous forme libre : 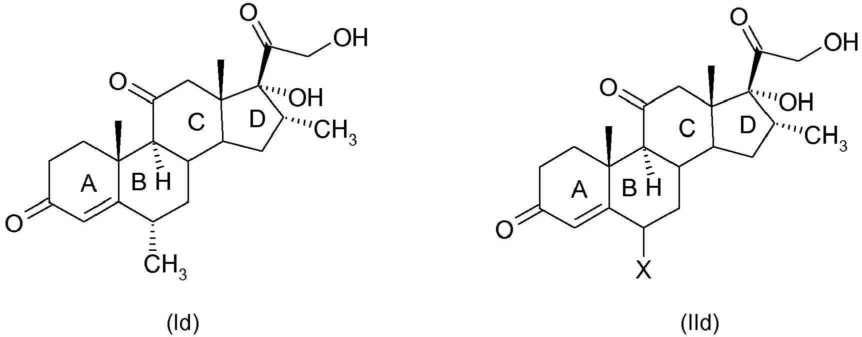 Ou comporter une protection sous forme d'un groupement oxa-spiranique (ici groupement 2, 4, 7, 9-oxaspiro [4.5] nonane).

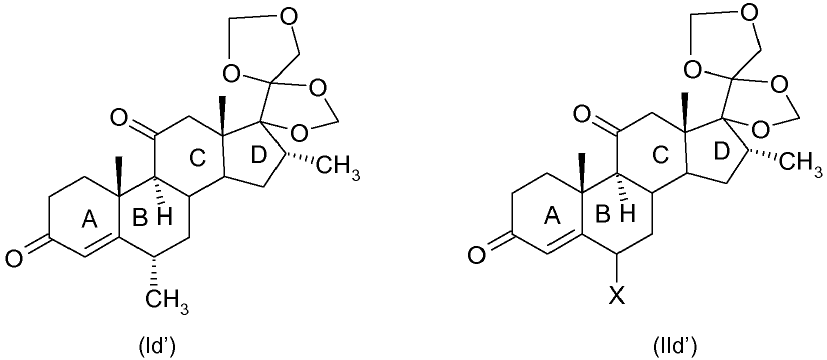

Dans cet exemple, on passe de la forme libre à la forme protégée par un traitement au formaldéhyde en présence d'un acide, selon des techniques connues.

Les composés (Id), (IId), (Id') et (IId') décrits ci-dessus sont des intermédiaires utiles pour la synthèse du cortivazol, corticosteroïde connu pour son effet anti-inflammatoire et immunosuppresseur. La synthèse de cortivazol à partir de composés sous forme libre respectivement de formule (Id) et (IId) ou protégée respectivement de formule (Id) ou (IId'), est par exemple décrite dans le brevet FR 1 590 064.

Par protection du groupement carboxyle, on entend groupement carboxyle estérifié, notamment un ester de C(1-6)alkyle, ou benzyle, ou allyle, ou ester de silyle, par exemple tréthylsilyle ou triméthylsylile.

Dans les procédés selon l'invention, l'étape d'alkylation du composé halogéné (II) peut se faire dans tout solvant approprié, par exemple le tétrahydrofurane, le 2-méthyle tetrahydrofurane, l'éther éthylique, le dioxane, préférentiellement à une température comprise entre -70 et 0 °C (-40°C en exemple).

Les agents d'alkylation peuvent éventuellement être préparés *in situ.* Par exemple, dans le cas des organocuprates, on peut faire réagir l'organolithien correspondant avec de l'iodure de cuivre.

Un avantage important des procédés d'alkylation selon l'invention, est qu'ils ne requièrent pas l'ajout d'un excès de cuivre. En effet, on obtient une alkylation complète dès un équivalent de cuivre par rapport au composé (II) à alkyler. Ainsi, les procédés selon l'invention fonctionnent avec une teneur réduite en métal.

Typiquement, la quantité de cuivre introduite est comprise entre 1 et 2 équivalents par rapport au composé (II) à alkyler, préférentiellement entre 1 et 1,5 équivalents, préférentiellement entre 1,1 et 1,3 équivalents, typiquement 1,2 équivalents.

La présente invention a donc pour objet un procédé de préparation d'un composé de formule (I) 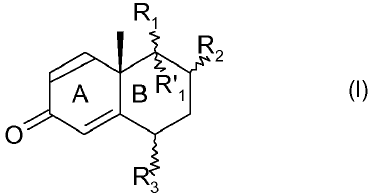
Où R₁, R₂ représentent indépendamment l'un de l'autre un atome d'hydrogène, un radical alkyle comprenant de 1 à 3 atomes de carbone, ou un atome d'halogène, ou bien R₁ et R₂ forment ensemble un cycle carboné comprenant de 4 à 6 atomes de carbone, ortho condensé au cycle B du composé (I), ledit cycle comprenant optionnellement une ou plusieurs doubles liaisons et étant optionnellement substitué par un ou plusieurs groupement alkyle comprenant 1 à 3 atomes de carbone, ou bien R₁ et R₂ forment ensemble les cycles C et D d'un squelette carboné stéroïdien, lesdits cycles C et D comprenant optionnellement une ou plusieurs doubles liaisons et étant optionnellement substitués par un ou plusieurs groupes choisis parmi les groupes alkyles linéaires ou ramifiés comprenant de 1 à 12 atomes de carbone, les groupes acyles comprenant de 1 à 12 atomes de carbone et optionnellement substitués par un ou plusieurs groupe hydroxyles, les groupements carboxyle, hydroxyle, ou oxo, sous forme libre ou protégée, ou les atomes d'halogènes, préférentiellement le fluor, chaque position desdits cycles C et D pouvant porter un ou, lorsque cela est possible, deux substituants,
R'₁ représente un atome d'hydrogène ou d'halogène, préférentiellement le fluor, et R₁ et R'₁ sont situés de part et d'autre du plan des cycles A et B,
R₃ représente un groupement alkyle comprenant de 1 à 4 atomes de carbone, préférentiellement un groupe méthyle, préférentiellement un groupe méthyle positionné en α,
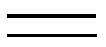 représente une simple ou une double liaison,
et 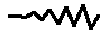 représente soit une simple liaison en alpha ou beta des cycles A et B, soit une double liaison dans le plan des cycles A et B,
comprenant une étape d'alkylation d'un composé de formule (II) 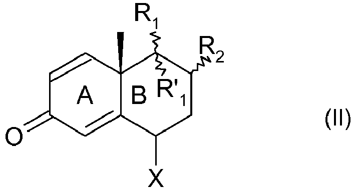
Où R₁, R'₁ et R₂, 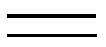 et 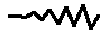 sont tels que définis dans le composé (I),
Où X est un atome d'halogène, préférentiellement le brome ou l'iode, préférentiellement le brome,
avec un agent d'alkylation organométallique permettant d'introduire un groupement alkyl R₃ en substitution de l'atome d'halogène X du composé (II), optionnellement en présence d'un catalyseur métallique, préférentiellement au palladium ou au cuivre, pour aboutir au composé de formule (I).

Selon un mode de réalisation, l'agent d'alkylation est choisi parmi les organolithiens de formule R₃Li, les organomagnésiens de formule R₃MgX', les organozinciques de formule R₃ZnX', où X' est un atome d'halogène, préférentiellement le chlore ou le brome, les organocuivreux de formule R₃Cu, les organocuprates lithiens de formule (R₃)₂CuLi ou les organocyanocuprates de formule R₃CuCNLi ou (R₃)₂CuCN(Li)₂, les halogénocuprates de formule R₃CuLiX", où X" est un halogène, préférentiellement l'iode ou le brome, les organoboroniques de formule R₃B(OH)₂, les organotrifluoroborates de formule R₃BF₃K, optionnellement en présence d'un catalyseur métallique, préférentiellement au nickel, au palladium ou au cuivre, préférentiellement au palladium ou au cuivre.

Selon un mode de réalisation particulier, l'agent d'alkylation est choisi parmi les organomagnésiens, les organocuivreux, les organocuprates lithiens, les organocyanocuprates, les halogénocuprates.

Selon un autre mode de réalisation, l'agent d'alkylation est un organomagnésien de formule R₃MgX', où X' est un atome d'halogène, préférentiellement le chlore ou le brome, préférentiellement le brome, et où l'alkylation est effectuée en présence d'un catalyseur au cuivre.

La présente invention a également pour objet un tel procédé où l'agent d'alkylation est un halogénocuprate de formule R₃CuLiX", où X" est un halogène, préférentiellement l'iode ou le brome.

La présente invention a également pour objet un tel procédé où l'agent d'alkylation est un organocuprate lithien de formule (R₃)₂CuLi ou un organocyanocuprate de formule (R₃)₂CuCN(Li)₂.

La présente invention a également pour objet un tel procédé où l'agent d'alkylation est un organocuprate lithien de formule (R₃)₂CuLi, et où l'alkylation comprend une étape de réaction du composé (II) avec ledit organocuprate lithien, suivie d'une réaction du composé ainsi obtenu avec un deuxième agent d'alkylation de formule R₃X"', où X'" est un atome d'halogène, préférentiellement l'iode.

Selon un mode de réalisation des procédés selon l'invention, le composé (II) est obtenu par halogénation d'un composé de formule (III) : 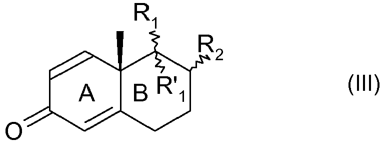 Où R₁, R'₁, R₂, 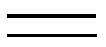 et 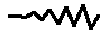 sont tels que définis plus haut,

Selon un mode de réalisation des procédés selon l'invention, X représente un atome de brome.

Selon un mode de réalisation des procédés selon l'invention, le composé (II) est obtenu par réaction du composé (III) avec un N-Bromoimide tel que le N-bromo-succinimide, en présence d'un initiateur radicalaire tel que le peroxyde de benzoyle ou l'azobisisobutyronitrile.

Selon un mode de réalisation des procédés selon l'invention, dans les composés de formule (I) et (II), R₁ et R₂ forment ensemble les cycles C et D d'un squelette carboné stéroïdien, lesdits cycles C et D comprenant optionnellement une ou plusieurs doubles liaisons et étant optionnellement substitués par un ou plusieurs groupes choisis parmi les groupes alkyles linéaires ou ramifiés comprenant de 1 à 12 atomes de carbone, les groupes acyles comprenant de 1 à 12 atomes de carbone et optionnellement substitués par un ou plusieurs groupe hydroxyles, les groupements carboxyle, hydroxyle, ou oxo, sous forme libre ou protégée, ou les atomes d'halogènes, préférentiellement le fluor, chaque position desdits cycles C et D pouvant porter un ou, lorsque cela est possible, deux substituants.

Selon un mode de réalisation des procédés selon l'invention, le composé (I) est un composé stéroïdien de formule (IA) : 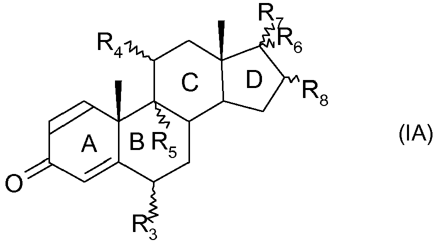
Où R₃ est un groupement alkyle comprenant de 1 à 4 atomes de carbone, préférentiellement un méthyle,
R₄ est soit l'hydrogène, soit un groupement hydroxyle ou oxo, sous forme libre ou protégée, par exemple un groupe hydroxyle sous forme estérifiée, préférentiellement sous forme acétylée ou sous forme trifluoroacétylée,
R₅ est soit l'hydrogène, soit un atome d'halogène, préférentiellement le fluor,
R₆ est soit l'hydrogène, soit un groupement hydroxyle, sous forme libre ou protégée,
R₇ est soit l'hydrogène, soit un groupement -C(O)R₇' ou -C(OR)₂R₇', où R est un groupement protecteur de la fonction carbonyle, et où R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée, préférentiellement un groupe hydroxyméthyle sous forme acétylée, ou sous forme trifluoroacétylée, ou bien R₆ et R₇ forment ensemble un groupement oxo en position 17 du squelette stéroïdien du composé (IA),
R₈ est soit l'hydrogène, soit un groupement alkyle comprenant de 1 à 3 atomes de carbone, où les groupements -R₆ et -R₇ sont situés de part et d'autre du plan des cycles A, B, C, D,
où 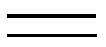 représente une simple ou une double liaison,
où 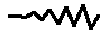 représente soit une simple liaison en alpha ou beta ou dans le plan des cycles A, B, C, D, soit, lorsque cela est possible, une double liaison dans le plan des cycles A, B, C, D.
et le composé (II) est un composé de formule (IIA) : 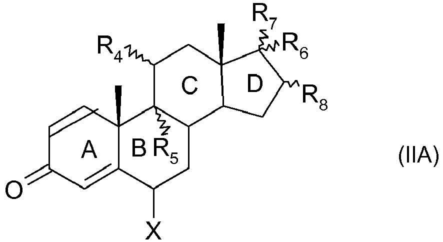
où R₄ et R₅, R₆, R₇, R₈ 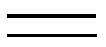 et 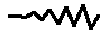 sont tels que définis dans le composé (IA),
où X est un atome d'halogène, préférentiellement le brome ou l'iode, préférentiellement le brome ;

Selon un mode de réalisation des procédés selon l'invention, le composé (I) est un composé stéroïdien de formule (IA') : 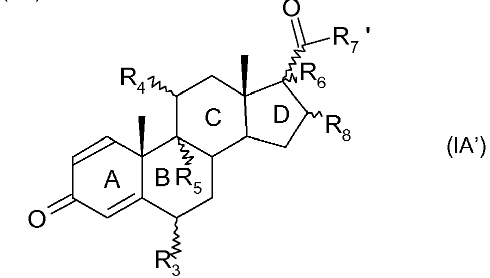
Où R₃ est un groupement alkyle comprenant de 1 à 4 atomes de carbone, préférentiellement un méthyle,
R₄ est soit l'hydrogène, soit un groupement hydroxyle ou oxo, sous forme libre ou protégée, par exemple un groupe hydroxyle sous forme estérifiée, préférentiellement sous forme acétylée, ou sous forme trifluoroacétylée.
R₅ est soit l'hydrogène, soit un atome d'halogène, préférentiellement le fluor,
R₆ est soit l'hydrogène, soit un groupement hydroxyle, sous forme libre ou protégée,
R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée, préférentiellement un groupe hydroxyméthyle sous forme acétylée, ou sous forme trifluoroacétylée,
R₈ est soit l'hydrogène, soit un groupement alkyle comprenant de 1 à 3 atomes de carbone, où les groupements -R₆ et -(CO)R₇' sont situés de part et d'autre du plan des cycles A, B, C, D,
où le groupement oxo situé sur le carbone en position 20 du squelette stéroïdien peut être sous forme libre ou protégée,
où 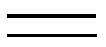 représente une simple ou une double liaison,
où 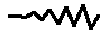 représente soit une simple liaison en alpha ou beta ou dans le plan des cycles A, B, C, D, soit, lorsque cela est possible, une double liaison dans le plan des cycles A, B, C, D.
et le composé (II) est un composé de formule (IIA') : 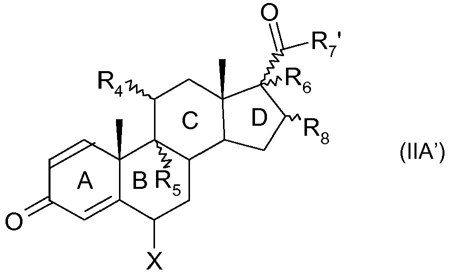
où R₄ et R₅, R₆, R₇', R₈ 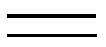 et 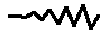 sont tels que définis dans le composé (IA'),
où le groupement oxo situé sur le carbone 20 du squelette stéroïdien peut être sous forme libre ou protégée,
où X est un atome d'halogène, préférentiellement le brome ou l'iode, préférentiellement le brome ;
Selon un mode de réalisation des procédés selon l'invention, le composé (I) est un composé de formule (IB) : 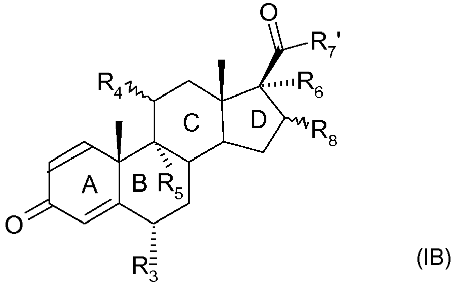
Où R₃ est un groupement alkyle comprenant de 1 à 4 atomes de carbone, préférentiellement un méthyle,
R₄ est soit l'hydrogène, soit un groupement hydroxyle en beta, sous forme libre ou protégée, par exemple un groupe hydroxyle sous forme estérifiée, préférentiellement sous forme acétylée ou sous forme trifluoroacétylée, soit un groupement oxo, sous forme libre ou protégée,
R₅ est soit l'hydrogène, soit un atome d'halogène, préférentiellement le fluor,
R₆ est soit l'hydrogène, soit un groupement hydroxyle, sous forme libre ou protégée, par exemple sous forme estérifiée,
R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée, préférentiellement un groupe hydroxyméthyle sous forme acétylée ou sous forme trifluoroacétylée,
R₈ est soit l'hydrogène, soit un groupement alkyle en alpha comprenant de 1 à 3 atomes de carbone,
où le groupement oxo situé sur le carbone 20 du squelette stéroïdien peut être sous forme libre ou protégée,
où 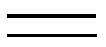 représente une simple ou une double liaison,
où 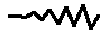 représente soit une simple liaison en alpha ou beta ou dans le plan des cycles A, B, C, D, soit, lorsque cela est possible, une double liaison dans le plan des cycles A, B, C, D.
et le composé (II) est un composé de formule (IIB) : 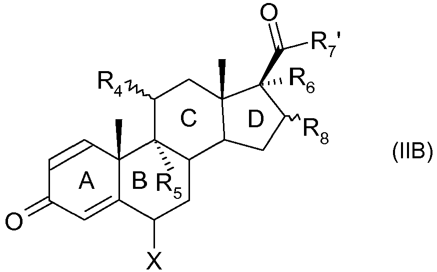
où R₄ et R₅, R₆, R₇', R₈, 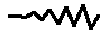 et 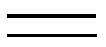 sont tels que définis dans le composé (IB),
où le groupement oxo situé sur le carbone 20 du squelette stéroïdien peut être sous forme libre ou protégée,
où X est un atome d'halogène, préférentiellement le brome ou l'iode, préférentiellement le brome.

Selon un mode de réalisation des procédés selon l'invention le composé (I) est un composé de formule (IC) : 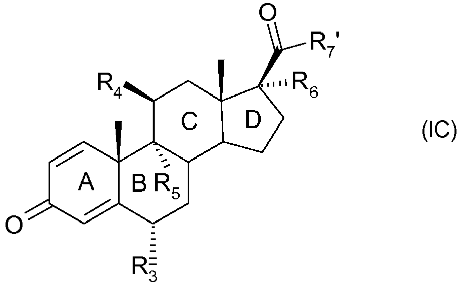
Où R₃ est un groupement alkyle comprenant de 1 à 4 atomes de carbone, préférentiellement un méthyle,
R₄ est soit l'hydrogène, soit un groupement hydroxyle, sous forme libre ou protégée, par exemple un groupe hydroxyle sous forme estérifiée, préférentiellement sous forme acétylée ou sous forme trifluoroacétylée,
R₅ est soit l'hydrogène, soit un atome d'halogène, préférentiellement le fluor,
R₆ est soit l'hydrogène, soit un groupement hydroxyle, sous forme libre ou protégée, par exemple sous forme estérifiée,
R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée, préférentiellement un groupe hydroxyméthyle sous forme acétylée, ou sous forme trifluoroacétylée,
et le composé (II) est un composé de formule (IIC) : 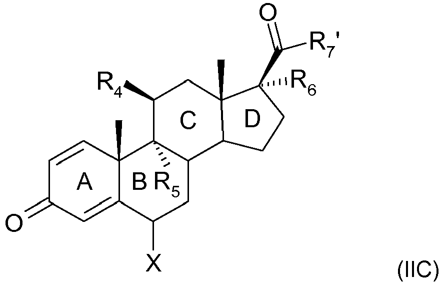
Où R₄ et R₅, R₆, R₇' sont tels que définis dans le composé (IC),
Où X est un atome d'halogène, préférentiellement le brome ou l'iode, préférentiellement le brome.

Selon un mode de réalisation des procédés selon l'invention, dans les composés (IC) et (IIC), R₇' est un groupement méthyle, ou un groupement hydroxyméthyle sous forme libre ou protégée, préférentiellement sous forme acétylée ou sous forme trifluoroacétylée.

Selon un mode de réalisation des procédés selon l'invention, dans les composés (IC) et (IIC), R₆ est un groupement hydroxyle, sous forme libre ou protégée.

Selon un mode de réalisation des procédés selon l'invention, dans les composés (IC) et (IIC), R₄ et R₇ sont respectivement des groupements hydroxyle et hydroxyméthyle sous forme protégée, préférentiellement sous forme estérifiée, préférentiellement sous forme acétylée ou sous forme trifluoroacétylée.

Selon un mode de réalisation des procédés selon l'invention, dans les composés (IC) et (IIC), R₅ est un atome d'hydrogène ou de fluor.

La présente demande décrit des composés de formule (IIC) Dans lequel : 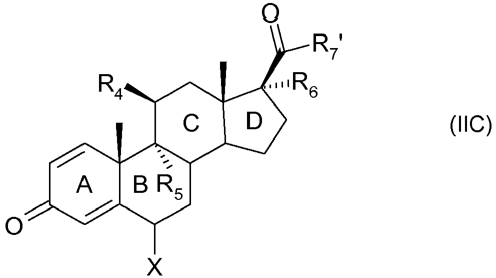
- X est un atome d'halogène, préférentiellement le brome,
- R₅ est un atome d'hydrogène ou de fluor,
- R₄ et R₇' sont respectivement des groupements hydroxyle et hydroxyméthyle sous forme protégée, préférentiellement sous forme estérifiée, préférentiellement sous forme acétylée ou sous forme trifluoroacétylée.
- R₆ est un groupement hydroxyle, sous forme libre ou protégée.

La présente demande décrit aussi des composés répondant à la formule (IIC) ci-dessus dans lesquels :
- X est un atome d'halogène, préférentiellement l'iode ou le brome, préférentiellement le brome
- R₅ est un atome d'hydrogène
- R₄ et R₇' sont respectivement des groupements hydroxyle et hydroxyméthyle sous forme acétylée ou sous forme trifluoroacétylée.
- R₆ est un groupement hydroxyle, sous forme libre ou protégée, préférentiellement sous forme libre

La présente invention a également pour objet des procédés tels que décrits précédemment où le composé (I) est un composé de formule (ID) : 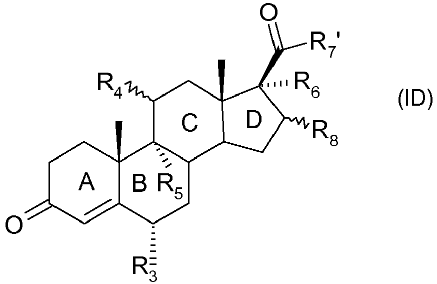
Où R₃ est un groupement alkyle comprenant de 1 à 4 atomes de carbone, préférentiellement un méthyle,
R₄ est soit l'hydrogène, soit un groupement hydroxyle en beta, sous forme libre ou protégée, soit un groupement oxo, sous forme libre ou protégée,
R₅ est soit l'hydrogène, soit un atome d'halogène, préférentiellement le fluor,
R₆ est soit l'hydrogène, soit un groupement hydroxyle, sous forme libre ou protégée
R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée,
R8 est soit l'hydrogène, soit un groupement alkyle en alpha comprenant de 1 à 3 atomes de carbone, préférentiellement un groupement méthyle,
où le groupement oxo situé sur le carbone 20 du squelette stéroïdien peut être sous forme libre ou protégée,
où 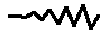 représente soit une simple liaison en alpha ou beta ou dans le plan des cycles A, B, C, D, soit, lorsque cela est possible, une double liaison dans le plan des cycles A, B, C, D.
et où le composé (II) est un composé de formule (IID) : 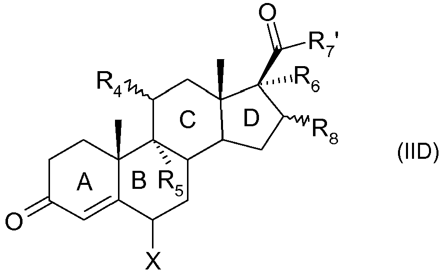
où R₄ et R₅, R₆, R₇', R₈, 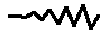 sont tels que définis dans le composé (ID),
où le groupement oxo situé sur le carbone 20 du squelette stéroïdien peut être sous forme libre ou protégée, de la même manière qu'il est présent dans le composé (ID)
où X est un atome d'halogène, préférentiellement le brome ou l'iode, préférentiellement le brome.

Selon un mode de réalisation de tels procédés, dans les composés (ID) et (IID), R₄ est soit un groupement hydroxyle en beta, sous forme libre ou protégée, soit un groupement oxo, sous forme libre ou protégée.

Selon un mode de réalisation de tels procédés, dans les composés (ID) et (IID), R₇' est un groupement méthyle, ou un groupement hydroxyméthyle sous forme libre ou protégée.

Selon un mode de réalisation de tels procédés, dans les composés (ID) et (IID), R₆ est un groupement hydroxyle, sous forme libre ou protégée.

Selon un mode de réalisation de tels procédés, dans les composés (ID) et (IID), R₅ est un atome d'hydrogène.

Selon un mode de réalisation de tels procédés, dans les composés (ID) et (IID), R₇' est un groupement hydroxyméthyle sous forme libre ou protégée.

Selon un mode de réalisation de tels procédés, dans les composés (ID) et (IID), R₈ est un groupement méthyle en alpha.

Selon un mode de réalisation de tels procédés, dans les composés (ID) et (IID), R₆ est un groupement hydroxyle, R₇' est un groupement hydroxyméthyle, et R₆, R₇', et le groupement oxo du carbone 20 du squelette stéroïdoien sont protégés conjointement sous forme d'un groupement oxa-spiranique.

Selon un mode de réalisation de tels procédés, les composés (Id') et (IId') ont pour formule respectivement : 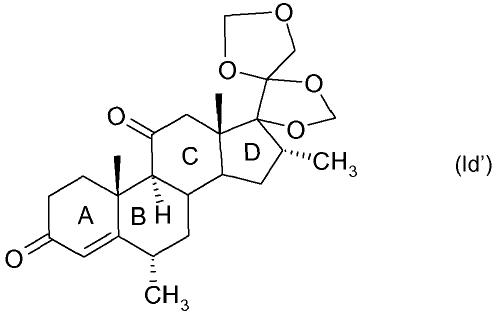 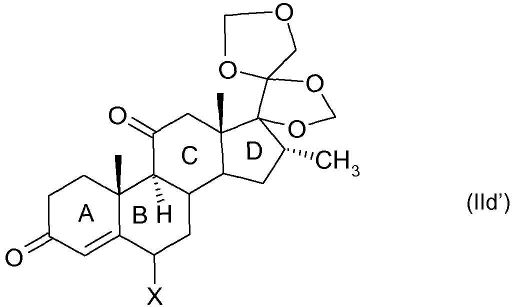 où X est un atome d'halogène, préférentiellement le brome ou l'iode, préférentiellement le brome.

La présente demande décrit des composés répondant à la formule (IId) ci-dessous : 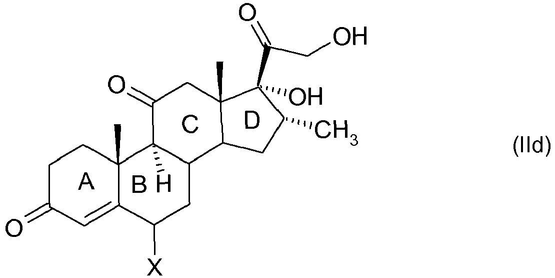 où X est un atome d'halogène, préférentiellement le brome ou l'iode, préférentiellement le brome.

La présente demande décrit des composés répondant à la formule (IId') ci-dessous : 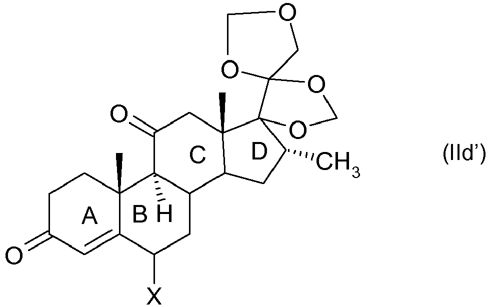 où X est un atome d'halogène, préférentiellement le brome ou l'iode, préférentiellement le brome.

### Exemples :

### Exemple 1: (4aS, 8R)-8-bromo-4α-methyl-5, 6, 7, 8, tetrahydronaphtalène-2(4αH)-one

Dans un ballon équipé d'une agitation magnétique, on a chargé 1gramme (6,17 mmol) de (S)-4α-methyl-5,6, 7, 8, tetrahydronaphtalène-2(4αH)-one dans 60 ml de tétrachlorométhane. Puis 1,65 grammes (9,25 mmol) de N bromosuccinimide et 0,30 gramme (1,23 moles) de peroxyde de benzoyle ont été ajoutés. La suspension résultante a été agitée sous reflux une heure. La solution orangée résultante a ensuite été refroidie à température ambiante et 100 ml de dichlorométhane ont été ajoutés. Le mélange réactionnel a ensuite été lavé avec une solution aqueuse saturée de bicarbonate de sodium. Les phases organiques ont ensuite été lavée à l'eau, déshydratées au sulfate de sodium, filtrées, et le solvant évaporé ; Le produit a ensuite été purifié sur colonne chromatographique sur gel de silice pour obtenir la (4aS, 8R)-8-bromo-4α-méthyl-5,6, 7, 8, tetrahydronaphtalène-2(4αH)-one. (0,6 gramme, 99% pureté).

**1H-NMR (CDCl3, 400 MHz,):** d (ppm), 6.73 (d, J=9.94 Hz, 1 H), 6.27 (d, J=1.95 Hz, 1 H), 6.20 (dd, J= 9.94, 1.75 Hz,1 H), 5.07 - 5.01 (m, 1 H), 2.35 (ddd, J= 15.01, 3.02, 2.83 Hz, 1 H), 2.30 - 2.17 (m, 1 H), 2.02 - 1.92 (m, 1 H), 1.92 -1.71 (m, 2 H), 1.62 (s, 3 H), 1.41 (td, J=13.45, 4.09 Hz, 1 H).
**13C-NMR (CDCl3, 100 MHz):** d (ppm), 186.44, 160.67, 158.08, 127.02, 125.62, 50.97, 40.42, 36.44, 36.05, 27.74, 16.51.

### Exemple 2: (4aS, 8R)-4α,8-diméthyl-5, 6, 7, 8, tetrahydronaphtalène-2(4αH)-one

Dans un vase Schlenk séché et purgé sous argon de 10 ml, équipé d'une agitation magnétique et d'un septum, on a chargé 104 mg d'iodure de cuivre dans le tétrahydrofurane sec (3 ml), et le mélange gris a été refroidi à 0°C. On a ensuite ajouté goutte à goutte 1,09 mmol de MeLi, toujours à 0°C, et le mélange jaune a été agité à cette température jusqu'à obtention d'une solution incolore. On forme ainsi in situ le dimethylcuprate lithié. La solution a été refroidie à -40°C, et une solution de 110 mg (0,45 mmol) de (4aS, 8R)-8-bromo-4α-methyl-5, 6, 7, 8, tetrahydronaphtalène-2(4αH)-one dans le tétrahydrofurane sec a été ajoutée goutte à goutte. Après agitation à -40°C, on ajoute 1,36 mmol d'iodure de méthyle, et le mélange orange est agité encore 30 minutes à cette température ; la réaction est ensuite stoppée avec une solution aqueuse de chlorure d'ammonium à 25%, et on extrait les produits de réaction à l'acétate d'éthyle ; les phases organiques sont ensuite lavées avec de la saumure, séchées au sulfate de sodium, filtrées et concentrées sous vide. Le résidu est ensuite purifié sur colonne chromatographique sur gel de silice pour obtenir la (4aS, 8R)-4α,8-dimethyl-5,6,7,8, tetrahydronaphtalène-2(4αH)-one ; (27 mg, 98% pureté).

### Exemple 3: (4aS, 8R)-4α,8-dimethyl-5,6,7,8, tetrahydronaphtalène-2(4αH)-one

Dans un vase Schlenk séché et purgé sous argon de 10 ml, équipé d'une agitation magnétique et d'un septum, on a chargé 104 mg d'iodure de cuivre dans le tétrahydrofurane sec (3 ml), et le mélange gris a été refroidi à 0°C. On a ensuite ajouté goutte à goutte 1,09 mmol de MeLi, toujours à 0°C, et le mélange jaune a été agité à cette température jusqu'à obtention d'une solution incolore. On forme ainsi in situ le dimethylcuprate lithié. La solution a été refroidie à -40°C, et une solution de 110 mg (0,45 mmol) de (4αS, 8R)-8-bromo-4α-methyl-5,6,7,8,tetrahydronaphtalène-2(4αH)-one dans le tétrahydrofurane sec a été ajoutée goutte à goutte. Après agitation 10 minutes à -40°C, la réaction est ensuite stoppée avec une solution aqueuse de chlorure d'ammonium à 25%, et on extrait les produits de réaction à l'acétate d'éthyle ; les phases organiques sont ensuite lavées avec de la saumure, séchées au sulfate de sodium, filtrées et concentrées sous vide. Le résidu est ensuite purifié sur colonne chromatographique sur gel de silice pour obtenir la (4aS, 8R)-4α,8-dimethyl-5,6,7,8, tetrahydronaphtalène-2(4αH)-one ; (28 mg, 98% pureté).

### Exemple 4: (4aS, 8R)-4α,8-dimethyl-5, 6, 7, 8, tetrahydronaphtalène-2(4αH)-one

Dans un vase Schlenk séché et purgé sous argon de 10 ml, équipé d'une agitation magnétique et d'un septum, on a chargé 104 mg d'iodure de cuivre dans le tétrahydrofurane sec (3 ml), et le mélange gris a été refroidi à 0°C. On a ensuite ajouté goutte à goutte 0,55 mmol de MeLi, toujours à 0°C, et le mélange jaune a été agité à cette température jusqu'à obtention d'une solution incolore. On forme ainsi in situ le mono méthyl cuprate lithié. La solution a été refroidie à -40°C, et une solution de 110 mg (0,45 mmol) de (4aS, 8R)-8-bromo-4α-methyl-5, 6, 7, 8, tetrahydronaphtalène-2(4αH)-one dans le tétrahydrofurane sec a été ajoutée goutte à goutte. Après agitation 10 minutes à -40°C, la réaction est ensuite stoppée avec une solution aqueuse de chlorure d'ammonium à 25%, et on extrait les produits de réaction à l'acétate d'éthyle ; les phases organiques sont ensuite lavées avec de la saumure, séchées au sulfate de sodium, filtrées et concentrées sous vide. Le résidu est ensuite purifié sur colonne chromatographique sur gel de silice pour obtenir la (4aS, 8R)-4α,8-dimethyl-5,6,7,8,tetrahydronaphtalène-2(4αH)-one ; (36 mg, 98% pureté).

**1H-NMR (CDCl3, 300 MHz):** d (ppm), 6.78 (d, J=9.95 Hz, 1 H), 6.21 (dd, J=9.81, 1.80 Hz, 1 H), 6.11 (t, J=1.66 Hz, 1H), 2.58 - 2.43 (m, 1 H), 2.04 - 1.93 (m, 1 H), 1.89 - 1.63 (m, 3 H), 1.36 -1.28 (m, 1 H), 1.25 (s, 3 H), 1.13 (d, J=6.63 Hz, 3 H), 1.03 (dd, J= 12.72, 4.42 Hz, 1 H). **13C-NMR (CDCl3, 75 MHz):** d (ppm), 187.41, 171.09, 158.08, 126.28, 121.41, 41.20, 38.55, 36.96, 34.09, 23.35, 20.98, 17.63.

### Exemple 5: diacétylation de la prednisolone, pour protéger les hydroxyles en 11 et 21

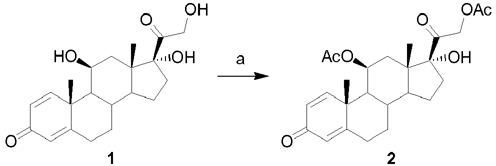

Dans un ballon de 500 mL, la prednisolone (21,60 g, 60 mmol) est introduite et mise en solution dans le dichlorométhane (300 mL). La DMAP (1,5 g, 12 mmol, 0,2 équiv), la triéthylamine (24,2 mL, 180 mmol, 3 équiv) et l'anhydride acétique (17,0 mL, 180 mmol, 3,0 équiv) sont ajoutés successivement dans le milieu réactionnel puis celui-ci est agité à 25 °C. L'avancement de la réaction est suivi par HPLC. Après 17 h d'agitation, la conversion de la prednisolone est complète. Le milieu réactionnel est quenché avec une solution saturée de chlorure d'ammonium puis la phase organique est extraite au dichlorométhane. La phase organique est séchée sur sulfate de magnésium puis concentrée sous pression réduite. Le brut est purifié par chromatographie sur gel de silice (dichlorométhane/méthanol 99 :1) pour donner le composé 2 avec un rendement de 90 % (pureté 98 %).

### Exemple 6: bromation en 6 du diacétate de prednisolone

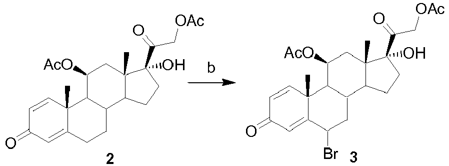

A une solution de la prednisolone diacétylée 2 (4,44 g, 10 mmol) dans le tétrachlorure de carbone (500 mL), le NBS (3,56 g, 20 mmol, 2,0 équiv) et le peroxyde de benzoyle (0,48 g, 2 mmol, 0,2 équiv) sont ajoutés. Le milieu réactionnel est porté à reflux et agité pendant environ 3 h. L'avancement de la réaction est contrôlé par HPLC. Au-delà de 3 h, la réaction n'évolue plus. Le milieu réactionnel est refroidi à température ambiante puis filtré. Le ballon est rincé 3 fois au tétrachlorure de carbone.

Le filtrat est ensuite concentré sous vide. Le brut est extrait au dichlorométhane et lavé avec une solution à 10 % d'hydrogénocarbonate de sodium. La phase organique est séchée sur sulfate de magnésium puis concentrée sous pression réduite. Le brut est purifié par chromatographie sur gel de silice (dichlorométhane/méthanol 99 :1) pour donner le composé 3 avec un rendement de 84 % (pureté 93 %).

Il est à noter qu'on peut également utiliser le chlorobenzène comme solvant pour cette synthèse.

### Exemple 7: 6α-methylprednisolone diacetate

Dans un vase Schlenk séché et purgé sous argon de 10 ml, équipé d'une agitation magnétique et d'un septum, on a chargé 228 mg d'iodure de cuivre dans le tetrahydrofurane sec (6 ml), et le mélange gris a été refroidi à 0°C. On a ensuite ajouté goutte à goutte 2,4 mmol de MeLi, toujours à 0°C, et le mélange jaune a été agité à cette température jusqu'à obtention d'une solution incolore. On forme ainsi in situ le diméthylcuprate lithié. La solution a été refroidie à -40°C, et une solution de 523 mg (1,0 mmol) de 6β-bromo-prednisolone diacetate dans le tétrahydrofurane sec (2ml) a été ajoutée goutte à goutte.

Après agitation 10 minutes à -40°C, la réaction est ensuite stoppée avec une solution aqueuse de chlorure d'ammonium à 25%, et on extrait les produits de réaction à l'acétate d'éthyle ; les phases organiques sont ensuite lavées avec de la saumure, séchées au sulfate de sodium, filtrées et concentrées sous vide. Le résidu est ensuite purifié sur colonne chromatographique sur gel de silice pour obtenir la 6α-methyl prednisolone diacetate (120 mg, pureté 85%).

### Exemple 8: 6α-methylprednisolone diacetate

### Dans un vase Schlenk séché et purgé sous argon de 10 ml, équipé d'une agitation magnétique et d'un septum, on a chargé 228 mg (1,2 mmol) d'iodure de cuivre dans le tetrahydrofurane sec (6 ml), et le mélange gris a été refroidi à 0°C. On a ensuite ajouté goutte à goutte 1,2 mmol de MeLi, toujours à 0°C, et le mélange jaune a été agité à cette température jusqu'à obtention d'une suspension orange. On forme ainsi in situ le mono méthyl cuprate. La solution a été refroidie à -40°C, et une solution de 523 mg (1,0 mmol) de 6β-bromo-prednisolone diacetate dans le tetrahydrofurane sec (2 ml) a été ajoutée goutte à goutte.

Après agitation 10 minutes à -40°C, la réaction est ensuite stoppée avec une solution aqueuse de chlorure d'ammonium à 25%, et on extrait les produits de réaction à l'acétate d'éthyle ; les phases organiques sont ensuite lavées avec de la saumure, séchées au sulfate de sodium, filtrées et concentrées sous vide. Le résidu est ensuite purifié sur colonne chromatographique sur gel de silice pour obtenir la 6α-methyl prednisolone diacetate (160 mg, pureté 98%).

**1H-NMR (CDCl3, 600 MHz):** d (ppm), 6.96 (d, J=9.98 Hz, 1 H), 6.27 (dd, J=9.98, 1.76 Hz, 1 H), 6.03 (d, J=1.76 Hz, 1H), 5.54 (d, J=2.93 Hz, 1 H), 5.09 (d, J=17.02 Hz, 1 H), 4.68 (d, *J*=17.61 Hz, 1 H), 2.79 - 2.73 (m, 1 H), 2.64 - 2.57(m, 1 H), 2.21 (qd, *J*=11.25, 4.40 Hz, 1 H), 2.15 (s, 3 H), 2.09 (s, 3 H), 2.06 - 2.01 (m, 2 H), 1.85 - 1.80 (m, 2 H),1.77 - 1.71 (m, 1 H), 1.54 - 1.41 (m, 2 H), 1.27 (s, 3 H), 1.21 - 1.15 (m, 1 H), 1.13 (d, *J*=6.46 Hz, 3 H), 0.94 - 0.85(m, 2 H), 0.84 (s, 3 H).

**13C-NMR (CDCl3, 150 MHz):** d (ppm), 204.64, 186.05, 171.99, 170.30, 169.66, 155.19, 127.67, 119.74, 89.07, 71.12, 67.45, 54.04, 50.81, 47.18, 43.06, 42.30, 35.24, 34.37, 32.86, 31.39, 23.50, 21.57, 20.90, 20.27, 17.47, 16.36.

## Revendications

1. Procédé de préparation d'un composé de formule (I) 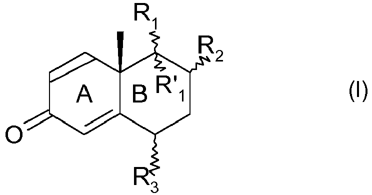
où R₁, R₂ représentent indépendamment l'un de l'autre un atome d'hydrogène, un radical alkyle comprenant de 1 à 3 atomes de carbone, ou un atome d'halogène, ou bien R₁ et R₂ forment ensemble un cycle carboné comprenant de 4 à 6 atomes de carbone, ortho condensé au cycle B du composé (I), ledit cycle comprenant optionnellement une ou plusieurs doubles liaisons et étant optionnellement substitué par un ou plusieurs groupement alkyle comprenant 1 à 3 atomes de carbone, ou bien R₁ et R₂ forment ensemble les cycles C et D d'un squelette carboné stéroïdien, lesdits cycles C et D comprenant optionnellement une ou plusieurs doubles liaisons et étant optionnellement substitués par un ou plusieurs groupes choisis parmi les groupes alkyles linéaires ou ramifiés comprenant de 1 à 12 atomes de carbone, les groupes acyles comprenant de 1 à 12 atomes de carbone et optionnellement substitués par un ou plusieurs groupe hydroxyles, les groupements carboxyle, hydroxyle, ou oxo, sous forme libre ou protégée, ou les atomes d'halogènes, préférentiellement le fluor, chaque position desdits cycles C et D pouvant porter un ou, lorsque cela est possible, deux substituants,
R'1 représente un atome d'hydrogène ou d'halogène, préférentiellement le fluor, et R₁ et R'₁ sont situés de part et d'autre du plan des cycles A et B,
R3 représente un groupement alkyle comprenant de 1 à 4 atomes de carbone, préférentiellement un groupe méthyle, préférentiellement un groupe méthyle positionné en α,
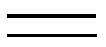 représente une simple ou une double liaison,
et 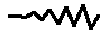 représente soit une simple liaison en alpha ou beta des cycles A et B, soit une double liaison dans le plan des cycles A et B,
comprenant une étape d'alkylation d'un composé de formule (II) 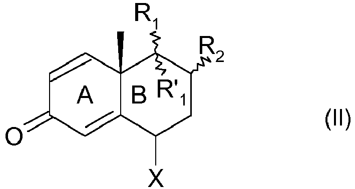
où R₁, R'₁ et R₂, 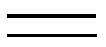 et 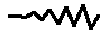 sont tels que définis dans le composé (I),
où X est un atome d'halogène, préférentiellement le brome ou l'iode, préférentiellement le brome,
avec un agent d'alkylation organométallique permettant d'introduire un groupement alkyl R₃ en substitution de l'atome d'halogène X du composé (II), optionnellement en présence d'un catalyseur métallique, préférentiellement au palladium ou au cuivre, pour aboutir au composé de formule (I).

2. Procédé selon la revendication 1 où l'agent d'alkylation est choisi parmi les organolithiens de formule R₃Li, les organomagnésiens de formule R₃MgX', les organozinciques de formule R₃ZnX', où X' est un atome d'halogène, préférentiellement le chlore ou le brome, les organocuivreux de formule R₃Cu, les organocuprates lithiens de formule (R₃)₂CuLi ou les organocyanocuprates de formule R₃CuCNLi ou (R₃)₂CuCN(Li)₂, les halogénocuprates de formule R₃CuLiX", où X" est un halogène, préférentiellement l'iode ou le brome, les organoboroniques de formule R₃B(OH)₂, les organotrifluoroborates de formule R₃BF₃K, optionnellement en présence d'un catalyseur métallique, préférentiellement au nickel, au palladium ou au cuivre, préférentiellement au palladium ou au cuivre.

3. Procédé selon la revendication 2 où l'agent d'alkylation est choisi parmi les organomagnésiens, les organocuivreux, les organocuprates lithiens, les organocyanocuprates, les halogénocuprates.

4. Procédé selon la revendication 3 où l'agent d'alkylation est un organomagnésien de formule R₃MgX', où X' est un atome d'halogène, préférentiellement le chlore ou le brome, préférentiellement le brome, et où l'alkylation est effectuée en présence d'un catalyseur au cuivre.

5. Procédé selon la revendication 3 où l'agent d'alkylation est un halogénocuprate de formule R₃CuLiX", où X" est un halogène, préférentiellement l'iode ou le brome.

6. Procédé selon la revendication 3 où l'agent d'alkylation est un organocuprate lithien de formule (R₃)₂CuLi ou un organocyanocuprate de formule (R₃)₂CuCN(Li)₂.

7. Procédé selon la revendication 3 où l'agent d'alkylation est un organocuprate lithien de formule (R₃)₂CuLi, et où l'alkylation comprend une étape de réaction du composé (II) avec ledit organocuprate lithien, suivie d'une réaction du composé ainsi obtenu avec un deuxième agent d'alkylation de formule R₃X"', où X'" est un atome d'halogène, préférentiellement l'iode.

8. Procédé selon l'une des revendications 1 à 7 où le composé (II) est obtenu par halogénation d'un composé de formule (III) : 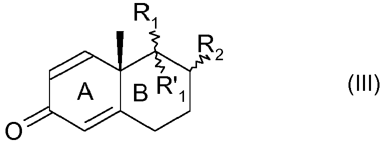 Où R₁, R'₁, R₂, 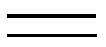 et 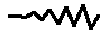 sont tels que définis à la revendication 1.

9. Procédé selon l'une des revendications 1 à 8 où X représente un atome de brome.

10. Procédé selon la revendication 8 et la revendication 9 où le composé (II) est obtenu par réaction du composé (III) avec un N-Bromoimide tel que le N-bromo-succinimide, en présence d'un initiateur radicalaire tel que le peroxyde de benzoyle ou l'azobisisobutyronitrile.

11. Procédé selon l'une des revendications 1 à 10 où, dans les composés de formule (I) et (II), R₁ et R₂ forment ensemble les cycles C et D d'un squelette carboné stéroïdien, lesdits cycles C et D comprenant optionnellement une ou plusieurs doubles liaisons et étant optionnellement substitués par un ou plusieurs groupes choisis parmi les groupes alkyles linéaires ou ramifiés comprenant de 1 à 12 atomes de carbone, les groupes acyles comprenant de 1 à 12 atomes de carbone et optionnellement substitués par un ou plusieurs groupe hydroxyles, les groupements carboxyle, hydroxyle, ou oxo, sous forme libre ou protégée, ou les atomes d'halogènes, préférentiellement le fluor, chaque position desdits cycles C et D pouvant porter un ou, lorsque cela est possible, deux substituants.

12. Procédé selon la revendication 11 où le composé (I) est un composé stéroïdien de formule (IA) : 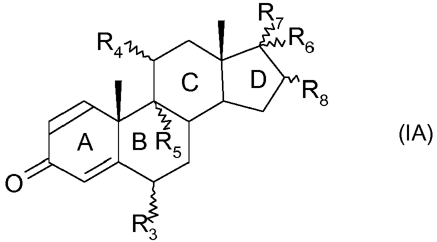
où R₃ est un groupement alkyle comprenant de 1 à 4 atomes de carbone, préférentiellement un méthyle,
R₄ est soit l'hydrogène, soit un groupement hydroxyle ou oxo, sous forme libre ou protégée, par exemple un groupe hydroxyle sous forme estérifiée, préférentiellement sous forme acétylée ou sous forme trifluoroacétylée,
R₅ est soit l'hydrogène, soit un atome d'halogène, préférentiellement le fluor,
R₆ est soit l'hydrogène, soit un groupement hydroxyle, sous forme libre ou protégée,
R₇ est soit l'hydrogène, soit un groupement -C(O)R₇' ou -C(OR)₂R₇', où R est un groupement protecteur de la fonction carbonyle, et où R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée, préférentiellement un groupe hydroxyméthyle sous forme acétylée, ou sous forme trifluoroacétylée, ou bien R₆ et R₇ forment ensemble un groupement oxo en position 17 du squelette stéroïdien du composé (IA),
R₈ est soit l'hydrogène, soit un groupement alkyle comprenant de 1 à 3 atomes de carbone, où les groupements -R₆ et -R₇ sont situés de part et d'autre du plan des cycles A, B, C, D,
où 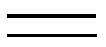 représente une simple ou une double liaison,
où 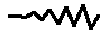 représente soit une simple liaison en alpha ou beta ou dans le plan des cycles A, B, C, D, soit, lorsque cela est possible, une double liaison dans le plan des cycles A, B, C, D.
et où le composé (II) est un composé de formule (IIA) : 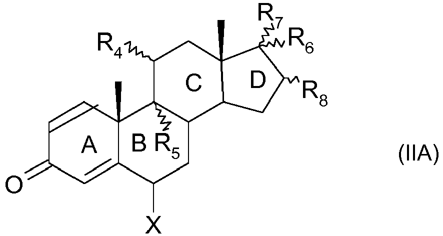
où R₄ et R₅, R₆, R₇, R₈ 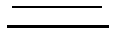 et 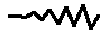 sont tels que définis dans le composé (IA),
où X est un atome d'halogène, préférentiellement le brome ou l'iode, préférentiellement le brome .

13. Procédé selon la revendication 12 où le composé (I) est un composé stéroïdien de formule (IA') : 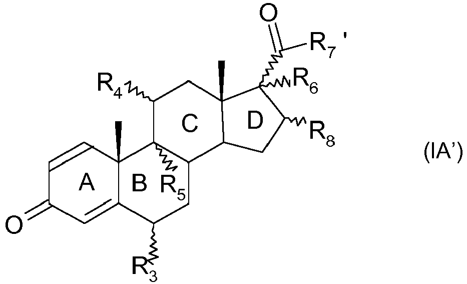
où R₃ est un groupement alkyle comprenant de 1 à 4 atomes de carbone, préférentiellement un méthyle,
R₄ est soit l'hydrogène, soit un groupement hydroxyle ou oxo, sous forme libre ou protégée, par exemple un groupe hydroxyle sous forme estérifiée, préférentiellement sous forme acétylée, ou sous forme trifluoroacétylée,
R₅ est soit l'hydrogène, soit un atome d'halogène, préférentiellement le fluor,
R₆ est soit l'hydrogène, soit un groupement hydroxyle, sous forme libre ou protégée,
R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée, préférentiellement un groupe hydroxyméthyle sous forme acétylée, ou sous forme trifluoroacétylée,
R₈ est soit l'hydrogène, soit un groupement alkyle comprenant de 1 à 3 atomes de carbone, où les groupements -R6 et -(CO)R7' sont situés de part et d'autre du plan des cycles A, B, C, D,
où le groupement oxo situé sur le carbone en position 20 du squelette stéroïdien peut être sous forme libre ou protégée,
où 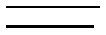 représente une simple ou une double liaison,
où 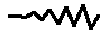 représente soit une simple liaison en alpha ou beta ou dans le plan des cycles A, B, C, D, soit, lorsque cela est possible, une double liaison dans le plan des cycles A, B, C, D.
et où le composé (II) est un composé de formule (IIA') : 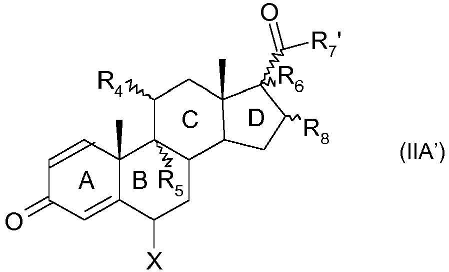
où R₄ et R₅, R₆, R₇', R₈ 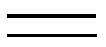 et 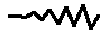 sont tels que définis dans le composé (IA'),
où le groupement oxo situé sur le carbone 20 du squelette stéroïdien peut être sous forme libre ou protégée,
où X est un atome d'halogène, préférentiellement le brome ou l'iode, préférentiellement le brome .

14. Procédé selon la revendication 13 où le composé (I) est un composé de formule (IB) : 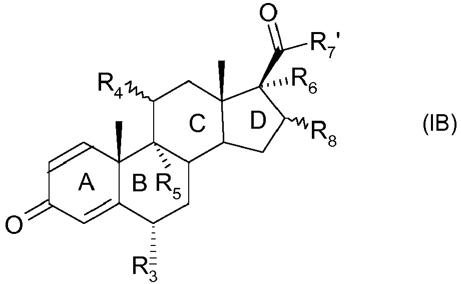
où R₃ est un groupement alkyle comprenant de 1 à 4 atomes de carbone, préférentiellement un méthyle,
R₄ est soit l'hydrogène, soit un groupement hydroxyle en beta, sous forme libre ou protégée, par exemple un groupe hydroxyle sous forme estérifiée, préférentiellement sous forme acétylée ou sous forme trifluoroacétylée, soit un groupement oxo, sous forme libre ou protégée,
R₅ est soit l'hydrogène, soit un atome d'halogène, préférentiellement le fluor,
R₆ est soit l'hydrogène, soit un groupement hydroxyle, sous forme libre ou protégée, par exemple sous forme estérifiée,
R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée, préférentiellement un groupe hydroxyméthyle sous forme acétylée ou sous forme trifluoroacétylée,
R₈ est soit l'hydrogène, soit un groupement alkyle en alpha comprenant de 1 à 3 atomes de carbone,
où le groupement oxo situé sur le carbone 20 du squelette stéroïdien peut être sous forme libre ou protégée,
où 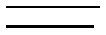 représente une simple ou une double liaison,
où 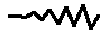 représente soit une simple liaison en alpha ou beta ou dans le plan des cycles A, B, C, D, soit, lorsque cela est possible, une double liaison dans le plan des cycles A, B, C, D.
et où le composé (II) est un composé de formule (IIB) : 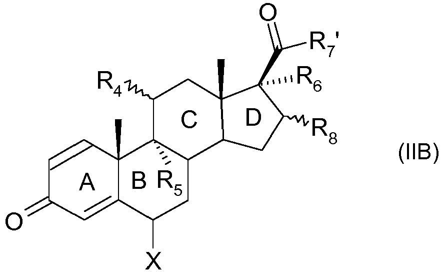
où R₄ et R₅, R₆, R₇', R₈, 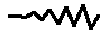 et 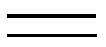 sont tels que définis dans le composé (IB),
où le groupement oxo situé sur le carbone 20 du squelette stéroïdien peut être sous forme libre ou protégée,
où X est un atome d'halogène, préférentiellement le brome ou l'iode, préférentiellement le brome.

15. Procédé selon la revendication 14 où le composé (I) est un composé de formule (IC) : 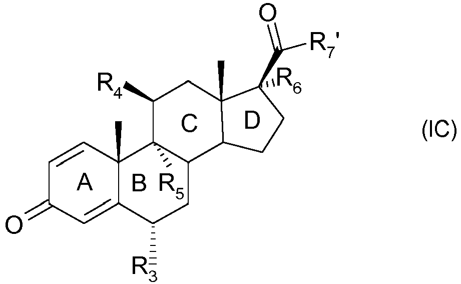
où R₃ est un groupement alkyle comprenant de 1 à 4 atomes de carbone, préférentiellement un méthyle,
R₄ est soit l'hydrogène, soit un groupement hydroxyle, sous forme libre ou protégée, par exemple un groupe hydroxyle sous forme estérifiée, préférentiellement sous forme acétylée ou sous forme trifluoroacétylée,
R₅ est soit l'hydrogène, soit un atome d'halogène, préférentiellement le fluor,
R₆ est soit l'hydrogène, soit un groupement hydroxyle, sous forme libre ou protégée, par exemple sous forme estérifiée,
R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée, préférentiellement un groupe hydroxyméthyle sous forme acétylée, ou sous forme trifluoroacétylée,
et où le composé (II) est un composé de formule (IIC) : 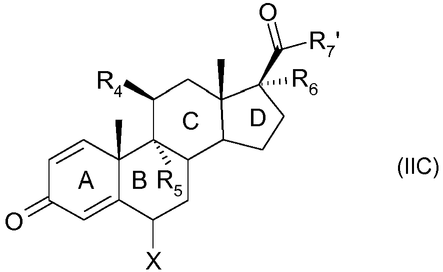
où R₄ et R₅, R₆, R₇' sont tels que définis dans le composé (IC),
où X est un atome d'halogène, préférentiellement le brome ou l'iode, préférentiellement le brome.

16. Procédé selon la revendication 15 où dans les composés (IC) et (IIC), R₇' est un groupement méthyle, ou un groupement hydroxyméthyle sous forme libre ou protégée, préférentiellement sous forme acétylée ou sous forme trifluoroacétylée.

17. Procédé selon l'une des revendications 15 ou 16 où dans les composés (IC) et (IIC), R₆ est un groupement hydroxyle, sous forme libre ou protégée.

18. Procédé selon l'une des revendications 15 à 17 où dans les composés (IC) et (IIC), R₄ et R₇ sont respectivement des groupements hydroxyle et hydroxyméthyle sous forme protégée, préférentiellement sous forme estérifiée, préférentiellement sous forme acétylée ou sous forme trifluoroacétylée.

19. Procédé selon l'une des revendications 15 à 18, où dans les composés (IC) et (IIC), R₅ est un atome d'hydrogène ou de fluor.

20. Procédé selon la revendication 13 où le composé (I) est un composé de formule (ID) : 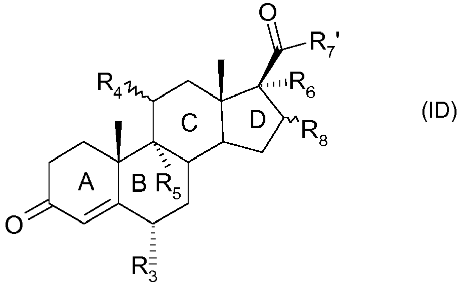
où R₃ est un groupement alkyle comprenant de 1 à 4 atomes de carbone, préférentiellement un méthyle,
R₄ est soit l'hydrogène, soit un groupement hydroxyle en beta, sous forme libre ou protégée, soit un groupement oxo, sous forme libre ou protégée,
R₅ est soit l'hydrogène, soit un atome d'halogène, préférentiellement le fluor,
R₆ est soit l'hydrogène, soit un groupement hydroxyle, sous forme libre ou protégée R₇' est soit l'hydrogène, soit un groupement alkyle comprenant de 1 à 3 atomes de carbone, soit un groupement hydroxyle sous forme libre ou protégée, soit un groupement hydroxyalkyle comprenant de 1 à 3 atomes de carbones sous forme libre ou protégée, préférentiellement un groupe hydroxyméthyle sous forme libre ou protégée,
R₈ est soit l'hydrogène, soit un groupement alkyle en alpha comprenant de 1 à 3 atomes de carbone, préférentiellement un groupement méthyle,
où le groupement oxo situé sur le carbone 20 du squelette stéroïdien peut être sous forme libre ou protégée,
où 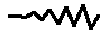 représente soit une simple liaison en alpha ou beta ou dans le plan des cycles A, B, C, D, soit, lorsque cela est possible, une double liaison dans le plan des cycles A, B, C, D.
et où le composé (II) est un composé de formule (IID) : 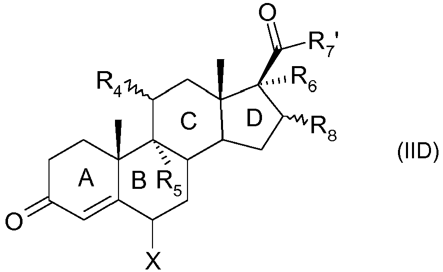
où R₄ et R₅, R₆, R₇', R₈, 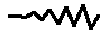 sont tels que définis dans le composé (ID),
où le groupement oxo situé sur le carbone 20 du squelette stéroïdien peut être sous forme libre ou protégée, de la même manière qu'il est présent dans le composé (ID) où X est un atome d'halogène, préférentiellement le brome ou l'iode, préférentiellement le brome.

21. Procédé selon la revendication 20 où dans les composés (ID) et (IID), R₄ est soit un groupement hydroxyle en beta, sous forme libre ou protégée, soit un groupement oxo, sous forme libre ou protégée.

22. Procédé selon l'une des revendications 20 ou 21 où dans les composés (ID) et (IID), R₇' est un groupement méthyle, ou un groupement hydroxyméthyle sous forme libre ou protégée.

23. Procédé selon l'une des revendications 20 à 22 où dans les composés (ID) et (IID), R₆ est un groupement hydroxyle, sous forme libre ou protégée.

24. Procédé selon l'une des revendications 20 à 23, où dans les composés (ID) et (IID), R₅ est un atome d'hydrogène.

25. Procédé selon l'une des revendications 20 à 24 où dans les composés (ID) et (IID), R₇' est un groupement hydroxyméthyle sous forme libre ou protégée.

26. Procédé selon l'une des revendications 20 à 25 où dans les composés (ID) et (IID), R₈ est un groupement méthyle en alpha.

27. Procédé selon l'une des revendications 20 à 26 où dans les composés (ID) et (IID), R₆ est un groupement hydroxyle, R₇' est un groupement hydroxyméthyle, et R₆, R₇', et le groupement oxo du carbone 20 du squelette stéroïdoien sont protégés conjointement sous forme d'un groupement oxa-spiranique.

28. Procédé selon la revendication 27 où les composés (ID) et (IID) correspondent aux composés respectivement de formule (Id') et (IId') : 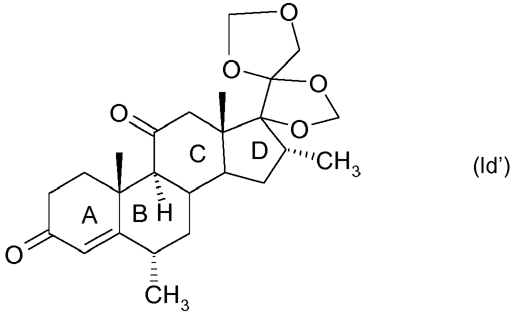 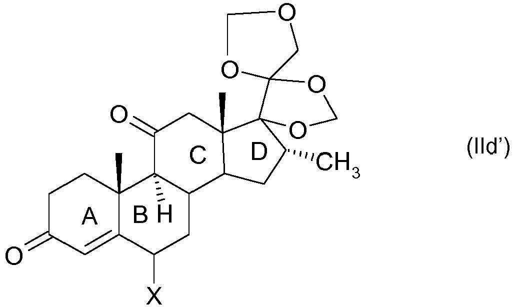 où X est un atome d'halogène, préférentiellement le brome ou l'iode, préférentiellement le brome.

## Patentansprüche

1. Verfahren zur Herstellung einer Verbindung der Formel (I) 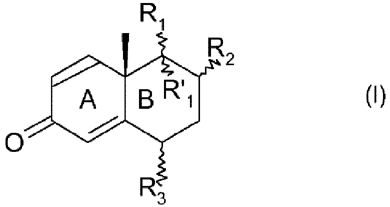
wobei R₁ und R₂ unabhängig voneinander für ein Wasserstoffatom, einen Alkylrest mit 1 bis 3 Kohlenstoffatomen oder ein Halogenatom stehen oder auch R₁ und R₂ zusammen einen Kohlenstoffring mit 4 bis 6 Kohlenstoffatomen bilden, der mit dem Ring B der Verbindung (I) ortho-anelliert ist, wobei der Ring gegebenenfalls eine oder mehrere Doppelbindungen enthält und gegebenenfalls durch eine oder mehrere Alkylgruppen mit 1 bis 3 Kohlenstoffatomen substituiert ist, oder auch R₁ und R₂ zusammen die Ringe C und D eines Steroid-Kohlenstoffgerüsts bilden, wobei die Ringe C und D gegebenenfalls eine oder mehrere Doppelbindungen enthalten und gegebenenfalls durch eine oder mehrere Gruppen, die aus linearen oder verzweigten Alkylgruppen mit 1 bis 12 Kohlenstoffatomen, Acylgruppen mit 1 bis 12 Kohlenstoffatomen, die gegebenenfalls durch eine oder mehrere Hydroxylgruppen substituiert sind, Carboxyl-, Hydroxyl- oder Oxogruppen in freier oder geschützter Form oder Halogenatomen, vorzugsweise Fluor, ausgewählt sind, substituiert sind, wobei jede Position der Ringe C und D einen oder, wenn dies möglich ist, zwei Substituenten tragen kann, R'₁ für ein Wasserstoff- oder Halogenatom, vorzugsweise Fluor, steht und R₁ und R'₁ sich auf der einen Seite und der anderen Seite der Ebene der Ringe A und B befinden,
R₃ für eine Alkylgruppe mit 1 bis 4 Kohlenstoffatomen, vorzugsweise eine Methylgruppe, vorzugsweise eine Methylgruppe in α-Position, steht,
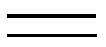 für eine Einfach- oder Doppelbindung steht und 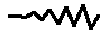 entweder für eine Einfachbindung in alpha- oder beta-Position der Ringe A und B oder für eine Doppelbindung in der Ebene der Ringe A und B steht,
umfassend einen Schritt der Alkylierung einer Verbindung der Formel (II) 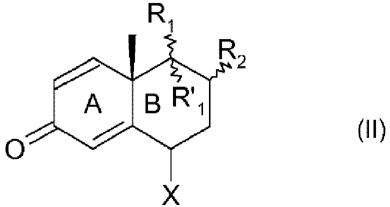
wobei R₁, R'₁ und R₂, 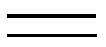 und 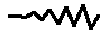 wie in Verbindung (I) definiert sind,
wobei X für ein Halogenatom, vorzugsweise Brom oder Iod, vorzugsweise Brom, steht,
mit einem metallorganischen Alkylierungsmittel, das die Einführung einer Alkylgruppe R₃ als Ersatz für das Halogenatom X der Verbindung (II) erlaubt, gegebenenfalls in Gegenwart eines Metallkatalysators, vorzugsweise eines Palladium- oder Kupferkatalysators, zu der Verbindung der Formel (I).

2. Verfahren nach Anspruch 1, wobei das Alkylierungsmittel aus Organolithiumverbindungen der Formel R₃Li, Organomagnesiumverbindungen der Formel R₃MgX', Organozinkverbindungen der Formel R₃ZnX', wobei X' für ein Halogenatom, vorzugsweise Chlor oder Brom, steht, Organokupferverbindungen der Formel R₃Cu, Lithiumorganocupraten der Formel (R₃)₂CuLi oder Organocyanocupraten der Formel R₃CuCNLi oder (R₃)₂CuCN(Li)₂, Halogenocupraten der Formel R₃CuLiX", wobei X" für ein Halogen, vorzugsweise Iod oder Brom, steht, Organoborverbindungen der Formel R₃B(OH)₂ und Organotrifluoroboraten der Formel R₃BF₃K, gegebenenfalls in Gegenwart eines Metallkatalysators, vorzugsweise eines Nickel-, Palladium- oder Kupferkatalysators, vorzugsweise eines Palladium- oder Kupferkatalysators, ausgewählt wird.

3. Verfahren nach Anspruch 2, wobei das Alkylierungsmittel aus Organomagnesiumverbindungen, Organokupferverbindungen, Lithiumorganocupraten, Organocyanocupraten und Halogenocupraten ausgewählt wird.

4. Verfahren nach Anspruch 3, wobei es sich bei dem Alkylierungsmittel um eine Organomagnesiumverbindung der Formel R₃MgX', wobei X' für ein Halogenatom, vorzugsweise Chlor oder Brom, vorzugsweise Brom, steht, handelt und dabei die Alkylierung in Gegenwart eines Kupferkatalysators durchgeführt wird.

5. Verfahren nach Anspruch 3, wobei es sich bei dem Alkylierungsmittel um ein Halogenocuprat der Formel R₃CuLiX", wobei X" für ein Halogen, vorzugsweise Iod oder Brom, steht, handelt.

6. Verfahren nach Anspruch 3, wobei es sich bei dem Alkylierungsmittel um ein Lithiumorganocuprat der Formel (R₃)₂CuLi oder ein Organocyanocuprat der Formel (R₃)₂CuCN(Li)₂ handelt.

7. Verfahren nach Anspruch 3, wobei es sich bei dem Alkylierungsmittel um ein Lithiumorganocuprat der Formel (R₃)₂CuLi handelt und wobei die Alkylierung einen Schritt der Umsetzung der Verbindung (II) mit dem Lithiumorganocuprat gefolgt von einer Umsetzung der so erhaltenen Verbindung mit einem zweiten Alkylierungsmittel der Formel R₃X"', wobei X"' für ein Halogenatom, vorzugsweise Iod, steht, umfasst.

8. Verfahren nach einem der Ansprüche 1 bis 7, wobei die Verbindung (II) durch Halogenierung einer Verbindung der Formel (III): 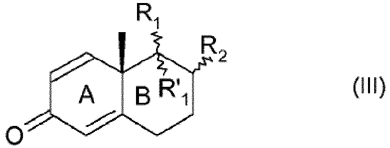
wobei R₁, R'₁, R₂, 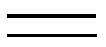 und 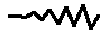 wie in Anspruch 1 definiert sind,
erhalten wird.

9. Verfahren nach einem der Ansprüche 1 bis 8, wobei X für ein Bromatom steht.

10. Verfahren nach Anspruch 8 und Anspruch 9, wobei die Verbindung (II) durch Umsetzung der Verbindung (III) mit einem N-Bromimid wie N-Bromsuccinimid in Gegenwart eines Radikalinitiators wie Benzoylperoxid oder Azobisisobutyronitril erhalten wird.

11. Verfahren nach einem der Ansprüche 1 bis 10, wobei in den Verbindungen der Formel (I) und (II) R₁ und R₂ zusammen die Ringe C und D eines Steroid-Kohlenstoffgerüsts bilden, wobei die Ringe C und D gegebenenfalls eine oder mehrere Doppelbindungen enthalten und gegebenenfalls durch eine oder mehrere Gruppen, die aus linearen oder verzweigten Alkylgruppen mit 1 bis 12 Kohlenstoffatomen, Acylgruppen mit 1 bis 12 Kohlenstoffatomen, die gegebenenfalls durch eine oder mehrere Hydroxylgruppen substituiert sind, Carboxyl-, Hydroxyl- oder Oxogruppen in freier oder geschützter Form oder Halogenatomen, vorzugsweise Fluor, ausgewählt sind, substituiert sind, wobei jede Position der Ringe C und D einen oder, wo dies möglich ist, zwei Substituenten tragen kann.

12. Verfahren nach Anspruch 11, wobei es sich bei der Verbindung (I) um eine Steroidverbindung der Formel (IA) handelt: 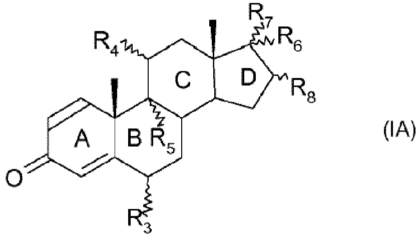
wobei R₃ für eine Alkylgruppe mit 1 bis 4 Kohlenstoffatomen, vorzugsweise ein Methyl, steht,
R₄ entweder für Wasserstoff oder für eine Hydroxyl- oder Oxogruppe in freier oder geschützter Form, beispielsweise eine Hydroxylgruppe in veresterter Form, vorzugsweise in acetylierter Form oder trifluoracetylierter Form, steht,
R₅ entweder für Wasserstoff oder für ein Halogenatom, vorzugsweise Fluor, steht,
R₆ entweder für Wasserstoff oder für eine Hydroxylgruppe in freier oder geschützter Form steht,
R₇ entweder für Wasserstoff oder für eine Gruppe -C(O)R₇' oder -C(OR)₂R₇' steht, wobei R für eine Schutzgruppe für die Carbonylfunktion steht und wobei R₇' entweder für Wasserstoff oder für eine Alkylgruppe mit 1 bis 3 Kohlenstoffatomen oder für eine Hydroxylgruppe in freier oder geschützter Form oder für eine Hydroxyalkylgruppe mit 1 bis 3 Kohlenstoffatomen in freier oder geschützter Form, vorzugsweise eine Hydroxymethylgruppe in freier oder geschützter Form, vorzugsweise eine Hydroxymethylgruppe in acetylierter Form oder in trifluoracetylierter Form, steht oder auch R₆ und R₇ zusammen eine Oxogruppe in der 17-Position des Steroidgerüsts der Verbindung (IA) bilden,
R₈ entweder für Wasserstoff oder für eine Alkylgruppe mit 1 bis 3 Kohlenstoffatomen steht, wobei die Gruppen -R₆ und -R₇ sich auf der einen Seite und der anderen Seite der Ebene der Ringe A, B, C und D befinden,
wobei 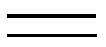 für eine Einfach- oder Doppelbindung steht,
wobei 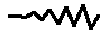 entweder für eine Einfachbindung in alpha- oder beta-Position oder in der Ebene der Ringe A, B, C und D oder, wenn dies möglich ist, für eine Doppelbindung in der Ebene der Ringe A, B, C und D steht,
und wobei es sich bei der Verbindung (II) um eine Verbindung der Formel (IIA) handelt: 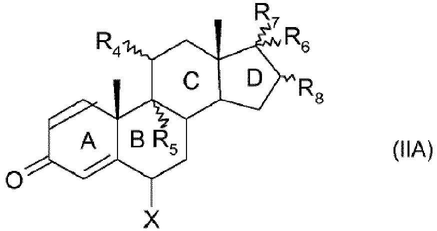
wobei R₄ und R₅, R₆, R₇, R₈, 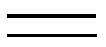 und 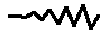 wie in Verbindung (IA) definiert sind,
wobei X für ein Halogenatom, vorzugsweise Brom oder Iod, vorzugsweise Brom, steht.

13. Verfahren nach Anspruch 12, wobei es sich bei der Verbindung (I) um eine Steroidverbindung der Formel (IA') handelt: 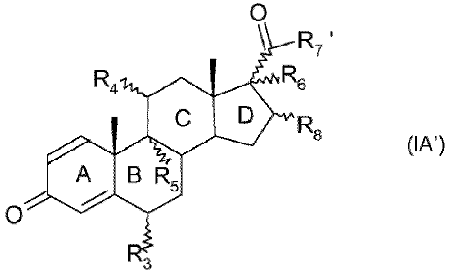
wobei R₃ für eine Alkylgruppe mit 1 bis 4 Kohlenstoffatomen, vorzugsweise ein Methyl, steht,
R₄ entweder für Wasserstoff oder für eine Hydroxyl- oder Oxogruppe in freier oder geschützter Form, beispielsweise eine Hydroxylgruppe in veresterter Form, vorzugsweise in acetylierter Form oder trifluoracetylierter Form, steht,
R₅ entweder für Wasserstoff oder für ein Halogenatom, vorzugsweise Fluor, steht,
R₆ entweder für Wasserstoff oder für eine Hydroxylgruppe in freier oder geschützter Form steht,
R₇' entweder für Wasserstoff oder für eine Alkylgruppe mit 1 bis 3 Kohlenstoffatomen oder für eine Hydroxylgruppe in freier oder geschützter Form oder für eine Hydroxyalkylgruppe mit 1 bis 3 Kohlenstoffatomen in freier oder geschützter Form, vorzugsweise eine Hydroxymethylgruppe in freier oder geschützter Form, vorzugsweise eine Hydroxymethylgruppe in acetylierter Form oder in trifluoracetylierter Form, steht,
R₈ entweder für Wasserstoff oder für eine Alkylgruppe mit 1 bis 3 Kohlenstoffatomen steht, wobei die Gruppen -R₆ und -(CO)R₇' sich auf der einen Seite und der anderen Seite der Ebene der Ringe A, B, C und D befinden,
wobei die Oxogruppe an dem Kohlenstoff in 20-Position des Steroidgerüsts in freier oder geschützter Form vorliegen kann,
wobei 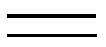 für eine Einfach- oder Doppelbindung steht,
wobei 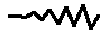 entweder für eine Einfachbindung in alpha- oder beta-Position oder in der Ebene der Ringe A, B, C und D oder, wenn dies möglich ist, für eine Doppelbindung in der Ebene der Ringe A, B, C und D steht,
und wobei es sich bei der Verbindung (II) um eine Verbindung der Formel (IIA') handelt: 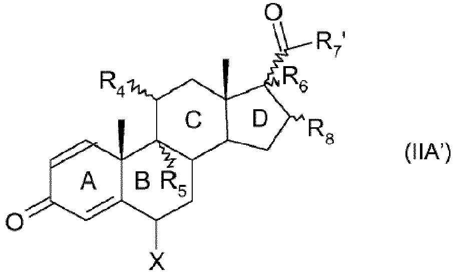
wobei R₄ und R₅, R₆, R₇', R₈, 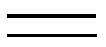 und 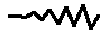 wie in Verbindung (IA') definiert sind,
wobei die Oxogruppe an dem 20-Kohlenstoff des Steroidgerüsts in freier oder geschützter Form vorliegen kann,
wobei X für ein Halogenatom, vorzugsweise Brom oder Iod, vorzugsweise Brom, steht.

14. Verfahren nach Anspruch 13, wobei es sich bei der Verbindung (I) um eine Verbindung der Formel (IB) handelt: 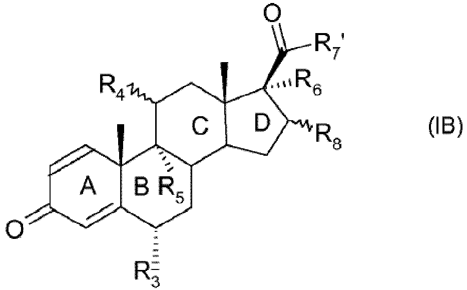
wobei R₃ für eine Alkylgruppe mit 1 bis 4 Kohlenstoffatomen, vorzugsweise ein Methyl, steht,
R₄ entweder für Wasserstoff oder für eine Hydroxylgruppe in beta-Position in freier oder geschützter Form, beispielsweise eine Hydroxylgruppe in veresterter Form, vorzugsweise in acetylierter Form oder trifluoracetylierter Form, oder für eine Oxogruppe in freier oder geschützter Form steht,
R₅ entweder für Wasserstoff oder für ein Halogenatom, vorzugsweise Fluor, steht,
R₆ entweder für Wasserstoff oder für eine Hydroxylgruppe in freier oder geschützter Form, beispielsweise in veresterter Form, steht,
R₇' entweder für Wasserstoff oder für eine Alkylgruppe mit 1 bis 3 Kohlenstoffatomen oder für eine Hydroxylgruppe in freier oder geschützter Form oder für eine Hydroxyalkylgruppe mit 1 bis 3 Kohlenstoffatomen in freier oder geschützter Form, vorzugsweise eine Hydroxymethylgruppe in freier oder geschützter Form, vorzugsweise eine Hydroxymethylgruppe in acetylierter Form oder in trifluoracetylierter Form, steht,
R₈ entweder für Wasserstoff oder für eine Alkylgruppe in alpha-Position mit 1 bis 3 Kohlenstoffatomen steht,
wobei die Oxogruppe an dem 20-Kohlenstoff des Steroidgerüsts in freier oder geschützter Form vorliegen kann,
wobei 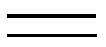 für eine Einfach- oder Doppelbindung steht,
wobei 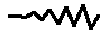 entweder für eine Einfachbindung in alpha- oder beta-Position oder in der Ebene der Ringe A, B, C und D oder, wenn dies möglich ist, für eine Doppelbindung in der Ebene der Ringe A, B, C und D steht,
und wobei es sich bei der Verbindung (II) um eine Verbindung der Formel (IIB) handelt: 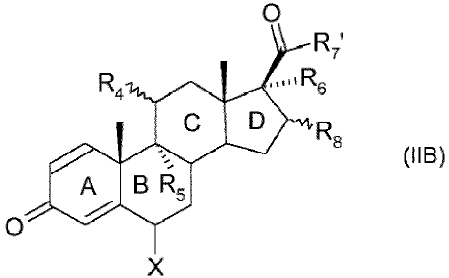
wobei R₄ und R₅, R₆, R₇', R₈, 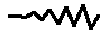 und 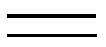 wie in Verbindung (IB) definiert sind,
wobei die Oxogruppe an dem 20-Kohlenstoff des Steroidgerüsts in freier oder geschützter Form vorliegen kann,
wobei X für ein Halogenatom, vorzugsweise Brom oder Iod, vorzugsweise Brom, steht.

15. Verfahren nach Anspruch 14, wobei es sich bei der Verbindung (I) um eine Verbindung der Formel (IC) handelt: 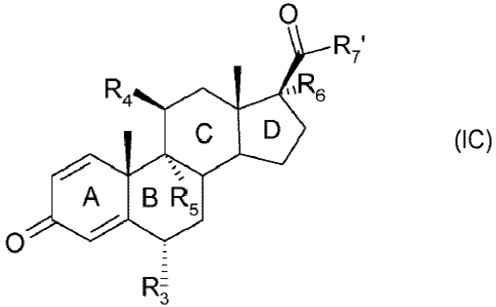
wobei R₃ für eine Alkylgruppe mit 1 bis 4 Kohlenstoffatomen, vorzugsweise ein Methyl, steht,
R₄ entweder für Wasserstoff oder für eine Hydroxylgruppe in freier oder geschützter Form, beispielsweise eine Hydroxylgruppe in veresterter Form, vorzugsweise in acetylierter Form oder in trifluoracetylierter Form, steht,
R₅ entweder für Wasserstoff oder für ein Halogenatom, vorzugsweise Fluor, steht,
R₆ entweder für Wasserstoff oder für eine Hydroxylgruppe in freier oder geschützter Form, beispielsweise in veresterter Form, steht,
R₇' entweder für Wasserstoff oder für eine Alkylgruppe mit 1 bis 3 Kohlenstoffatomen oder für eine Hydroxylgruppe in freier oder geschützter Form oder für eine Hydroxyalkylgruppe mit 1 bis 3 Kohlenstoffatomen in freier oder geschützter Form, vorzugsweise eine Hydroxymethylgruppe in freier oder geschützter Form, vorzugsweise eine Hydroxymethylgruppe in acetylierter Form oder in trifluoracetylierter Form, steht,
und wobei es sich bei der Verbindung (II) um eine Verbindung der Formel (IIC) handelt: 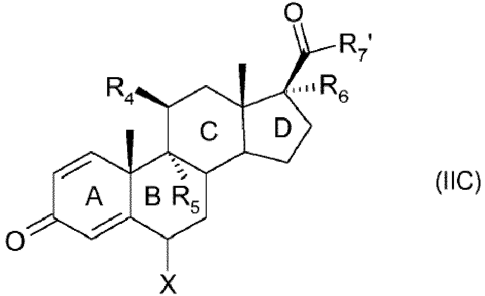
wobei R₄ und R₅, R₆, R₇' wie in Verbindung (IC) definiert sind,
wobei X für ein Halogenatom, vorzugsweise Brom oder Iod, vorzugsweise Brom, steht.

16. Verfahren nach Anspruch 15, wobei in den Verbindungen (IC) und (IIC) R₇' für eine Methylgruppe oder eine Hydroxymethylgruppe in freier oder geschützter Form, vorzugsweise in acetylierter Form oder in trifluoracetylierter Form, steht.

17. Verfahren nach Anspruch 15 oder 16, wobei in den Verbindungen (IC) und (IIC) R₆ für eine Hydroxylgruppe in freier oder geschützter Form steht.

18. Verfahren nach einem der Ansprüche 15 bis 17, wobei in den Verbindungen (IC) und (IIC) R₄ und R₇ jeweils für Hydroxyl- und Hydroxymethylgruppen in geschützter Form, vorzugsweise in veresterter Form, vorzugsweise in acetylierter Form oder in trifluoracetylierter Form, stehen.

19. Verfahren nach einem der Ansprüche 15 bis 18, wobei in den Verbindungen (IC) und (IIC) R₅ für ein Wasserstoff- oder Fluoratom steht.

20. Verfahren nach Anspruch 13, wobei es sich bei der Verbindung (I) um eine Verbindung der Formel (ID) handelt: 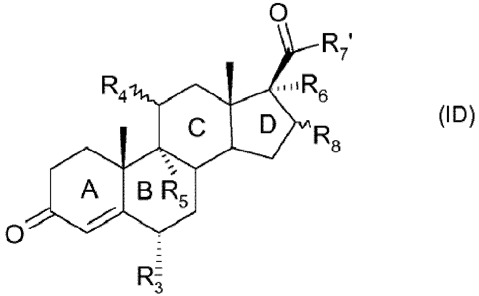
wobei R₃ für eine Alkylgruppe mit 1 bis 4 Kohlenstoffatomen, vorzugsweise ein Methyl, steht,
R₄ entweder für Wasserstoff oder für eine Hydroxylgruppe in beta-Position in freier oder geschützter Form, oder für eine Oxogruppe in freier oder geschützter Form steht,
R₅ entweder für Wasserstoff oder für ein Halogenatom, vorzugsweise Fluor, steht,
R₆ entweder für Wasserstoff oder für eine Hydroxylgruppe in freier oder geschützter Form steht,
R₇' entweder für Wasserstoff oder für eine Alkylgruppe mit 1 bis 3 Kohlenstoffatomen oder für eine Hydroxylgruppe in freier oder geschützter Form oder für eine Hydroxyalkylgruppe mit 1 bis 3 Kohlenstoffatomen in freier oder geschützter Form, vorzugsweise eine Hydroxymethylgruppe in freier oder geschützter Form, steht,
R₈ entweder für Wasserstoff oder für eine Alkylgruppe in alpha-Position mit 1 bis 3 Kohlenstoffatomen, vorzugsweise eine Methylgruppe, steht,
wobei die Oxogruppe an dem 20-Kohlenstoff des Steroidgerüsts in freier oder geschützter Form vorliegen kann,
wobei 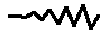 entweder für eine Einfachbindung in alpha- oder beta-Position oder in der Ebene der Ringe A, B, C und D oder, wenn dies möglich ist, für eine Doppelbindung in der Ebene der Ringe A, B, C und D steht,
und wobei es sich bei der Verbindung (II) um eine Verbindung der Formel (IID) handelt: 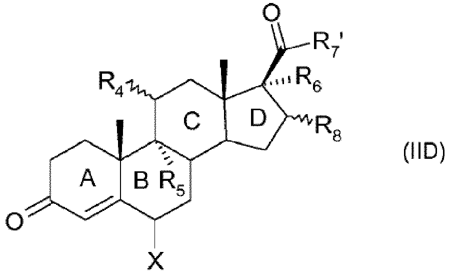
wobei R₄ und R₅, R₆, R₇', R₈ und 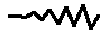 wie in Verbindung (ID) definiert sind,
wobei die Oxogruppe an dem 20-Kohlenstoff des Steroidgerüsts in freier oder geschützter Form vorliegen kann, und zwar ebenso, wie sie in der Verbindung (ID) vorliegt,
wobei X für ein Halogenatom, vorzugsweise Brom oder Iod, vorzugsweise Brom, steht.

21. Verfahren nach Anspruch 20, wobei in den Verbindungen (ID) und (IID) R₄ entweder für eine Hydroxylgruppe in beta-Position in freier oder geschützter Form oder für eine Oxogruppe in freier oder geschützter Form steht.

22. Verfahren nach Anspruch 20 oder 21, wobei in den Verbindungen (ID) und (IID) R₇' für eine Methylgruppe oder eine Hydroxymethylgruppe in freier oder geschützter Form steht.

23. Verfahren nach einem der Ansprüche 20 bis 22, wobei in den Verbindungen (ID) und (IID) R₆ für eine Hydroxylgruppe in freier oder geschützter Form steht.

24. Verfahren nach einem der Ansprüche 20 bis 23, wobei in den Verbindungen (ID) und (IID) R₅ für ein Wasserstoffatom steht.

25. Verfahren nach einem der Ansprüche 20 bis 24, wobei in den Verbindungen (ID) und (IID) R₇' für eine Hydroxymethylgruppe in freier oder geschützter Form steht.

26. Verfahren nach einem der Ansprüche 20 bis 25, wobei in den Verbindungen (ID) und (IID) R₈ für eine Methylgruppe in alpha-Position steht.

27. Verfahren nach einem der Ansprüche 20 bis 26, wobei in den Verbindungen (ID) und (IID) R₆ für eine Hydroxylgruppe steht, R₇' für eine Hydroxymethylgruppe steht und R₆, R₇' und die Oxogruppe des 20-Kohlenstoffs des Steroidgerüsts gemeinsam in Form einer Oxaspirangruppe geschützt sind.

28. Verfahren nach Anspruch 27, wobei die Verbindungen (ID) und (IID) den Verbindungen der Formel (Id') bzw. (IId') entsprechen: 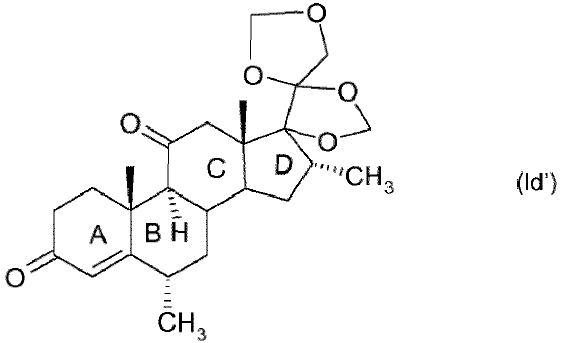 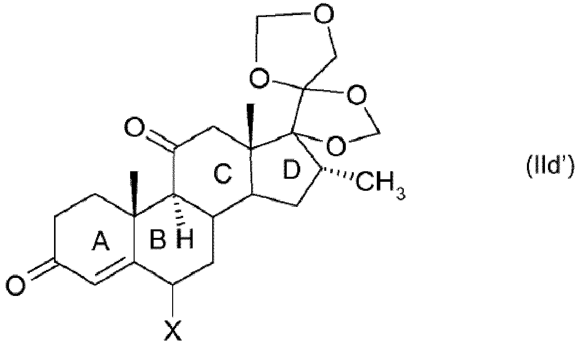 wobei X für ein Halogenatom, vorzugsweise Brom oder Iod, vorzugsweise Brom, steht.

## Claims

1. Process for preparing a compound of formula (I) 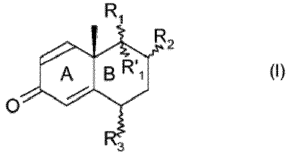
wherein R₁ and R₂ represent, independently of one another, a hydrogen atom, an alkyl radical comprising from 1 to 3 carbon atoms, or a halogen atom, or else R₁ and R₂ together form a carbon-based ring comprising from 4 to 6 carbon atoms, ortho-fused to the B ring of the compound (I), said ring optionally comprising one or more double bonds and being optionally substituted with one or more alkyl groups comprising 1 to 3 carbon atoms, or else R₁ and R₂ together form the C and D rings of a steroidal carbon-based backbone, said C and D rings optionally comprising one or more double bonds and being optionally substituted with one or more groups chosen from linear or branched alkyl groups comprising from 1 to 12 carbon atoms, acyl groups comprising from 1 to 12 carbon atoms and optionally substituted with one or more hydroxyl groups, carboxyl, hydroxyl or oxo groups, in free or protected form, or halogen atoms, preferentially fluorine, it being possible for each position of said C and D rings to bear one or, when this is possible, two substituents,
R'₁ represents a hydrogen or halogen atom, preferentially fluorine, and R₁ and R'₁ are located on either side of the plane of the A and B rings,
R₃ represents an alkyl group comprising from 1 to 4 carbon atoms, preferentially a methyl group, preferentially a methyl group positioned in the α position,
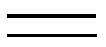 represents a single bond or a double bond,
and 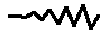 represents either a single bond in the α or β position of the A and B rings, or a double bond in the plane of the A and B rings,
comprising a step of alkylation of a compound of formula (II) 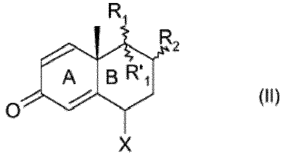
wherein R₁, R'₁ and R₂, 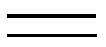 and 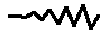 are as defined in the compound (I),
wherein X is a halogen atom, preferentially bromine or iodine, preferentially bromine,
with an organometallic alkylating agent which makes it possible to introduce an alkyl group R₃ as a replacement for the halogen atom X of the compound (II), optionally in the presence of a metal catalyst, preferentially a palladium or copper catalyst, so as to result in the compound of formula (I).

2. Process according to Claim 1, wherein the alkylating agent is chosen from organolithium compounds of formula R₃Li, organomagnesium compounds of formula R₃MgX', organozinc compounds of formula R₃ZnX', wherein X' is a halogen atom, preferentially chlorine or bromine, organocopper compounds of formula R₃Cu, lithium organocuprates of formula (R₃)₂CuLi or organocyanocuprates of formula R₃CuCNLi or (R₃)₂CuCN(Li)₂, halocuprates of formula R₃CuLiX", wherein X" is a halogen, preferentially iodine or bromine, organoboron compounds of formula R₃B(OH)₂, organotrifluoroborates of formula R₃BF₃K, optionally in the presence of a metal catalyst, preferentially a nickel, palladium or copper catalyst, preferentially a palladium or copper catalyst.

3. Process according to Claim 2, wherein the alkylating agent is chosen from organomagnesium compounds, organocopper compounds, lithium organocuprates, organocyanocuprates and halocuprates.

4. Process according to Claim 3, wherein the alkylating agent is an organomagnesium compound of formula R₃MgX', wherein X' is a halogen atom, preferentially chlorine or bromine, preferentially bromine, and wherein the alkylation is carried out in the presence of a copper catalyst.

5. Process according to Claim 3, wherein the alkylating agent is a halocuprate of formula R₃CuLiX", wherein X" is a halogen, preferentially iodine or bromine.

6. Process according to Claim 3, wherein the alkylating agent is a lithium organocuprate of formula (R₃)₂CuLi or an organocyanocuprate of formula (R₃)₂CuCN(Li)₂.

7. Process according to Claim 3, wherein the alkylating agent is a lithium organocuprate of formula (R₃)₂CuLi, and wherein the alkylation comprises a step of reacting the compound (II) with said lithium organocuprate, followed by a reaction of the compound thus obtained with a second alkylating agent of formula R₃X"', wherein X"' is a halogen atom, preferentially iodine.

8. Process according to one of Claims 1 to 7, wherein the compound (II) is obtained by halogenation of a compound of formula (III): 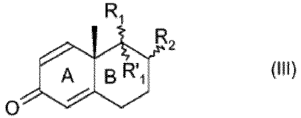 wherein R₁, R'₁, R₂, 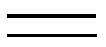 and 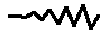 are as defined in Claim 1.

9. Process according to one of Claims 1 to 8, wherein X represents a bromine atom.

10. Process according to Claim 8 and Claim 9, wherein the compound (II) is obtained by reacting the compound (III) with an N-bromoimide, such as N-bromosuccinimide, in the presence of a radical initiator, such as benzoyl peroxide or azobisisobutyronitrile.

11. Process according to one of Claims 1 to 10, wherein, in the compounds of formulae (I) and (II), R₁ and R₂ together form the C and D rings of a steroidal carbon-based backbone, said C and D rings optionally comprising one or more double bonds and being optionally substituted with one or more groups chosen from linear or branched alkyl groups comprising from 1 to 12 carbon atoms, acyl groups comprising from 1 to 12 carbon atoms and optionally substituted with one or more hydroxyl groups, carboxyl, hydroxyl or oxo groups, in free or protected form, or halogen atoms, preferentially fluorine, it being possible for each position of said C and D rings to bear one or, when this is possible, two substituents.

12. Process according to Claim 11, wherein the compound (I) is a steroidal compound of formula (IA): 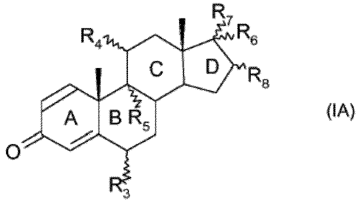
wherein R₃ is an alkyl group comprising from 1 to 4 carbon atoms, preferentially a methyl,
R₄ is either hydrogen, or a hydroxyl or oxo group, in free or protected form, for example a hydroxyl group in esterified form, preferentially in acetylated form or in trifluoroacetylated form,
R₅ is either hydrogen, or a halogen atom, preferentially fluorine,
R₆ is either hydrogen, or a hydroxyl group, in free or protected form,
R₇ is either hydrogen, or a -C(O)R₇' or -C(OR)₂R₇' group, wherein R is a protective group for the carbonyl function, and wherein R₇' is either hydrogen, or an alkyl group comprising from 1 to 3 carbon atoms, or a hydroxyl group in free or protected form, or a hydroxyalkyl group comprising from 1 to 3 carbon atoms, in free or protected form, preferentially a hydroxymethyl group in free or protected form, preferentially a hydroxymethyl group in acetylated form, or in trifluoroacetylated form, or else R₆ and R₇ together form an oxo group in the 17 position of the steroidal backbone of the compound (IA),
R₈ is either hydrogen, or an alkyl group comprising from 1 to 3 carbon atoms,
wherein the -R₆ and -R₇ groups are located on either side of the plane of the A, B, C and D rings,
wherein 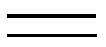 represents a single bond or a double bond, wherein 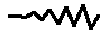 represents either a single bond in the α or β position or in the plane of the A, B, C and D rings, or, when this is possible, a double bond in the plane of the A, B, C and D rings,
and wherein the compound (II) is a compound of formula (IIA): 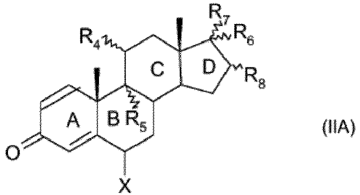
wherein R₄ and R₅, R₆, R₇, R₈, 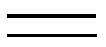 and 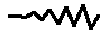 are as defined in the compound (IA),
wherein X is a halogen atom, preferentially bromine or iodine, preferentially bromine.

13. Process according to Claim 12, wherein the compound (I) is a steroidal compound of formula (IA'): 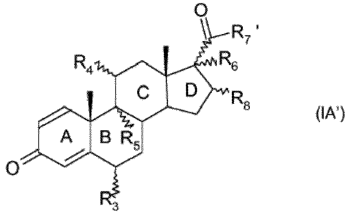
wherein R₃ is an alkyl group comprising from 1 to 4 carbon atoms, preferentially a methyl,
R₄ is either hydrogen, or a hydroxyl or oxo group, in free or protected form, for example a hydroxyl group in esterified form, preferentially in acetylated form, or in trifluoroacetylated form,
R₅ is either hydrogen, or a halogen atom, preferentially fluorine,
R₆ is either hydrogen, or a hydroxyl group, in free or protected form,
R₇' is either hydrogen, or an alkyl group comprising from 1 to 3 carbon atoms, or a hydroxyl group in free or protected form, or a hydroxyalkyl group comprising from 1 to 3 carbon atoms, in free or protected form, preferentially a hydroxymethyl group in free or protected form, preferentially a hydroxymethyl group in acetylated form, or in trifluoroacetylated form,
R₈ is either hydrogen, or an alkyl group comprising from 1 to 3 carbon atoms,
wherein the -R₆ and -(CO)R₇' groups are located on either side of the plane of the A, B, C and D rings,
wherein the oxo group located on the carbon in the 20 position of the steroidal backbone can be in free or protected form,
wherein 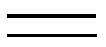 represents a single bond or a double bond, wherein 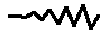 represents either a single bond in the α or β position or in the plane of the A, B, C and D rings, or, when this is possible, a double bond in the plane of the A, B, C and D rings,
and wherein the compound (II) is a compound of formula (IIA'): 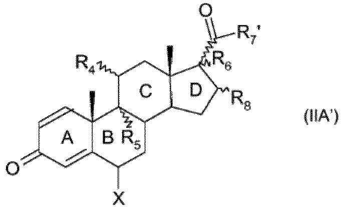
wherein R₄ and R₅, R₆, R₇', R₈, 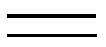 and 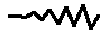 are as defined in the compound (IA'),
wherein the oxo group located on the carbon in the 20 position of the steroidal backbone can be in free or protected form,
wherein X is a halogen atom, preferentially bromine or iodine, preferentially bromine.

14. Process according to Claim 13, wherein the compound (I) is a compound of formula (IB): 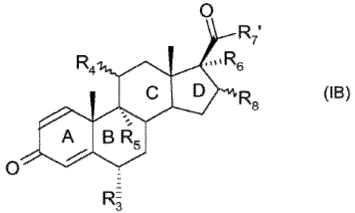
wherein R₃ is an alkyl group comprising from 1 to 4 carbon atoms, preferentially a methyl,
R₄ is either hydrogen, or a hydroxyl group in the β position, in free or protected form, for example a hydroxyl group in esterified form, preferentially in acetylated form or in trifluoroacetylated form, or an oxo group, in free or protected form,
R₅ is either hydrogen, or a halogen atom, preferentially fluorine,
R₆ is either hydrogen, or a hydroxyl group, in free or protected form, for example in esterified form,
R₇' is either hydrogen, or an alkyl group comprising from 1 to 3 carbon atoms, or a hydroxyl group in free or protected form, or a hydroxyalkyl group comprising from 1 to 3 carbon atoms, in free or protected form, preferentially a hydroxymethyl group in free or protected form, preferentially a hydroxymethyl group in acetylated form or in trifluoroacetylated form,
R₈ is either hydrogen, or an α-alkyl group comprising from 1 to 3 carbon atoms,
wherein the oxo group located on the carbon in the 20 position of the steroidal backbone can be in free or protected form,
wherein 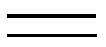 represents a single bond or a double bond, wherein 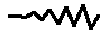 represents either a single bond in the α or β position or in the plane of the A, B, C and D rings, or, when this is possible, a double bond in the plane of the A, B, C and D rings,
and wherein the compound (II) is a compound of formula (IIB) : 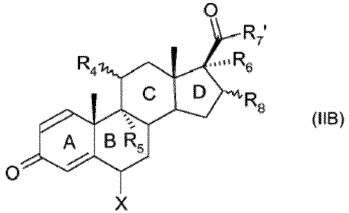
wherein R₄ and R₅, R₆, R₇', R₈, 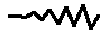 and 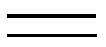 are as defined in the compound (IB),
wherein the oxo group located on the carbon in the 20 position of the steroidal backbone can be in free or protected form,
wherein X is a halogen atom, preferentially bromine or iodine, preferentially bromine.

15. Process according to Claim 14, wherein the compound (I) is a compound of formula (IC): 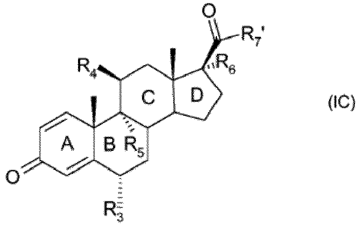
wherein R₃ is an alkyl group comprising from 1 to 4 carbon atoms, preferentially a methyl,
R₄ is either hydrogen, or a hydroxyl group, in free or protected form, for example a hydroxyl group in esterified form, preferentially in acetylated form or in trifluoroacetylated form,
R₅ is either hydrogen, or a halogen atom, preferentially fluorine,
R₆ is either hydrogen, or a hydroxyl group, in free or protected form, for example in esterified form,
R₇' is either hydrogen, or an alkyl group comprising from 1 to 3 carbon atoms, or a hydroxyl group in free or protected form, or a hydroxyalkyl group comprising from 1 to 3 carbon atoms, in free or protected form, preferentially a hydroxymethyl group in free or protected form, preferentially a hydroxymethyl group in acetylated form, or in trifluoroacetylated form,
and wherein the compound (II) is a compound of formula (IIC) : 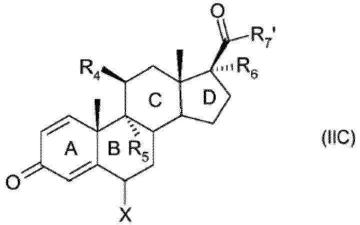
wherein R₄ and R₅, R₆ and R₇' are as defined in the compound (IC),
wherein X is a halogen atom, preferentially bromine or iodine, preferentially bromine.

16. Process according to Claim 15, wherein, in the compounds (IC) and (IIC), R₇' is a methyl group, or a hydroxymethyl group in free or protected form, preferentially in acetylated form or in trifluoroacetylated form.

17. Process according to either of Claims 15 and 16, wherein, in the compounds (IC) and (IIC), R₆ is a hydroxyl group, in free or protected form.

18. Process according to one of Claims 15 to 17, wherein, in the compounds (IC) and (IIC), R₄ and R₇ are respectively hydroxyl and hydroxymethyl groups in protected form, preferentially in esterified form, preferentially in acetylated form or in trifluoroacetylated form.

19. Process according to one of Claims 15 to 18, wherein, in the compounds (IC) and (IIC), R₅ is a hydrogen or fluorine atom.

20. Process according to Claim 13, wherein the compound (I) is a compound of formula (ID): 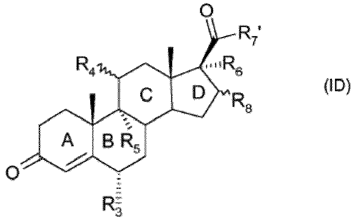
wherein R₃ is an alkyl group comprising from 1 to 4 carbon atoms, preferentially a methyl,
R₄ is either hydrogen, or a β-hydroxyl group, in free or protected form, or an oxo group, in free or protected form,
R₅ is either hydrogen, or a halogen atom, preferentially fluorine,
R₆ is either hydrogen, or a hydroxyl group, in free or protected form,
R₇' is either hydrogen, or an alkyl group comprising from 1 to 3 carbon atoms, or a hydroxyl group in free or protected form, or a hydroxyalkyl group comprising from 1 to 3 carbon atoms, in free or protected form, preferentially a hydroxymethyl group in free or protected form,
R₈ is either hydrogen, or an α-alkyl group comprising from 1 to 3 carbon atoms, preferentially a methyl group, wherein the oxo group located on the carbon in the 20 position of the steroidal backbone can be in free or protected form,
wherein 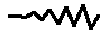 represents either a single bond in the α or β position or in the plane of the A, B, C and D rings, or, when this is possible, a double bond in the plane of the A, B, C and D rings,
and wherein the compound (II) is a compound of formula (IID) : 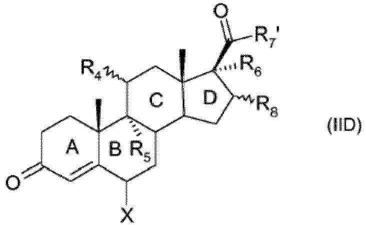
wherein R₄ and R₅, R₆, R₇', R₈ and 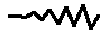 are as defined in the compound (ID),
wherein the oxo group located on the carbon in the 20 position of the steroidal backbone can be in free or protected form, in the same way as it is present in the compound (ID),
wherein X is a halogen atom, preferentially bromine or iodine, preferentially bromine.

21. Process according to Claim 20, wherein, in the compounds (ID) and (IID), R₄ is either a β-hydroxyl group, in free or protected form, or an oxo group, in free or protected form.

22. Process according to either of Claims 20 and 21, wherein, in the compounds (ID) and (IID), R₇' is a methyl group, or a hydroxymethyl group in free or protected form.

23. Process according to one of Claims 20 to 22, wherein, in the compounds (ID) and (IID), R₆ is a hydroxyl group, in free or protected form.

24. Process according to one of Claims 20 to 23, wherein, in the compounds (ID) and (IID), R₅ is a hydrogen atom.

25. Process according to one of Claims 20 to 24, wherein, in the compounds (ID) and (IID), R₇' is a hydroxymethyl group, in free or protected form.

26. Process according to one of Claims 20 to 25, wherein, in the compounds (ID) and (IID), R₈ is an α-methyl group.

27. Process according to one of Claims 20 to 26, wherein, in the compounds (ID) and (IID), R₆ is a hydroxyl group, R₇' is a hydroxymethyl group, and R₆, R₇', and the oxo group of the carbon in the 20 position of the steroidal backbone are jointly protected in the form of an oxaspirane group.

28. Process according to Claim 27, wherein the compounds (ID) and (IID) correspond to the compounds respectively of formulae (Id') and (IId'): 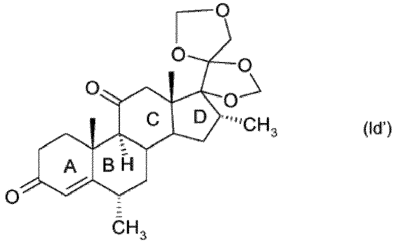 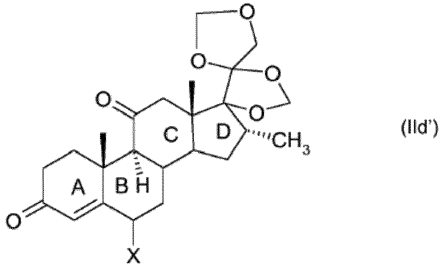 wherein X is a halogen atom, preferentially bromine or iodine, preferentially bromine.
